# EUROPEAN PATENT SPECIFICATION

(11) **EP 3 686 601 B1**
(45) Date of publication and mention of the grant of the patent: **10.05.2023**
(21) Application number: 20153426.0
(22) Date of filing: 23.01.2020
(51) Int. Cl.: G01N 33/569, C07K 14/445

(54) **SEROLOGICAL DETECTION OF PLASMODIUM ANTIBODIES**
SEROLOGISCHE DETEKTION VON PLASMODIUM-ANTIKÖRPERN
DÉTECTION SÉROLOGIQUE D'ANTICORPS DE PLASMODIUM

(30) Priority: 24.01.2019 EP 19153611
(43) Date of publication of application: 29.07.2020
(73) Proprietor: Euroimmun Medizinische Labordiagnostika AG, 23560 Lübeck (DE)
(72) Inventor: Deerberg, Andrea, 23627 Groß Grönau (DE); Probst, Christian, 23909 Ratzeburg (DE); Steinhagen, Katja, 23627 Groß Grönau (DE); Böthfür, Jana, 19217 Schlagsdorf (DE); Kruse, Tobias, 19217 Königsfeld/OT Klein Rünz (DE); Klemens, Oliver, 23562 Lübeck (DE); Menge, Babett, 21369 Nahrendorf (DE); Unger, Mandy, 20255 Hamburg (DE)

(56) References cited:
- EP-A2- 2 393 824
- US-A1- 2009 226 445
- FENG QIAN ET AL: "Immunogenicity of Self-Associated Aggregates and Chemically Cross-Linked Conjugates of the 42 kDa Plasmodium falciparum Merozoite Surface Protein-1", PLOS ONE, vol. 7, no. 6, 4 June 2012 (2012-06-04), page e36996, XP055697373, DOI: 10.1371/journal.pone.0036996
- SCOTT MUERHOFF A ET AL: "Detection of Plasmodium falciparum, P. vivax, P. ovale, and P. malariae merozoite surface protein 1-p19 antibodies in human malaria patients and experimentally infected nonhuman primates", CLINICAL AND DIAGNOSTIC LABORATORY IMMUNOLOGY, AMERICAN SOCIETY FOR MICROBIOLOGY, US, vol. 17, no. 10, 1 October 2010 (2010-10-01), pages 1631-1638, XP002640817, ISSN: 1556-6811, DOI: 10.1128/CVI.00196-10 [retrieved on 2010-08-11]
- JEFFREY W. PRIEST ET AL: "Specificity of the IgG antibody response to Plasmodium falciparum, Plasmodium vivax, Plasmodium malariae, and Plasmodium ovale MSP119 subunit proteins in multiplexed serologic assays", MALARIA JOURNAL, vol. 17, no. 1, 1 December 2018 (2018-12-01), XP055576143, DOI: 10.1186/s12936-018-2566-0
- KAE PUSIC ET AL: "Blood stage merozoite surface protein conjugated to nanoparticles induce potent parasite inhibitory antibodies", VACCINE, ELSEVIER, AMSTERDAM, NL, vol. 29, no. 48, 16 September 2011 (2011-09-16), pages 8898-8908, XP028326019, ISSN: 0264-410X, DOI: 10.1016/J.VACCINE.2011.09.070 [retrieved on 2011-09-21]
- JAIRO ANDRES FONSECA ET AL: "A chimeric protein-based malaria vaccine candidate induces robust T cell responses against Plasmodium vivax MSP119", SCIENTIFIC REPORTS, vol. 6, no. 1, 1 December 2016 (2016-12-01), XP055697010, DOI: 10.1038/srep34527
- AAKANKSHA KALRA ET AL: "Antigenicity of a Bacterially Expressed Triple Chimeric Antigen of Plasmodium falciparum AARP, MSP-311 and MSP-119: PfAMSP-Fu35", PLOS ONE, vol. 11, no. 10, 31 October 2016 (2016-10-31), page e0165720, XP055697147, DOI: 10.1371/journal.pone.0165720
- KAE M. PUSIC ET AL: "T Cell Epitope Regions of the P. falciparum MSP1-33 Critically Influence Immune Responses and In Vitro Efficacy of MSP1-42 Vaccines", PLOS ONE, vol. 6, no. 9, 13 September 2011 (2011-09-13), page e24782, XP055697148, DOI: 10.1371/journal.pone.0024782

## Description

The present invention relates to a polypeptide according to claim 1, a diagnostically useful carrier comprising such a polypeptide, an *in-vitro* method according to claim 5, a kit comprising the polypeptide and a use of the polypeptide for the manufacture of an analytical device, or for the manufacture of a composition, kit or reagent for detecting a *Malaria* infection.

Malaria is a mosquito-borne disease caused by a parasite. Four species of malaria parasites can infect humans under natural conditions: *Plasmodium falciparum, P. vivax, P. ovale* and *P*. *malariae.* The first two species cause the most infections worldwide. *P. falciparum* is the agent of severe, potentially fatal malaria, causing an estimated 700,000 - 2.7 million deaths annually. *P. vivax* and *P*. *ovale* have dormant liver stage parasites referred to as hypnozoites which can reactivate and cause malaria several months or years after the infecting mosquito bite. *P*. *malariae* produces long-lasting infections and, if left untreated, can persist asymptomatically in the human host for years, even a lifetime.

More recently, *P. knowlesi,* whose natural hosts include crab-eating macaques (*Macaca fascicularis*) and pigtailed macaques, has been shown to infect humans and may be responsible for a significant number of human infections in Malaysia.

In humans, the parasites grow and multiply first in the liver cells and then in the red blood cells of the blood. In the blood, successive broods of parasites grow inside the red blood cells and destroy them, releasing daughter parasites that continue the cycle by infecting cells.

Malaria must be recognized promptly in order to treat the patient in time and to prevent further spread of infection in the community. Malaria should be considered a potential medical emergency and should be treated accordingly.

Although uncomplicated malaria is quite treatable and the symptoms are non-disabling, severe malaria occurs when *P*. *falciparum* infections are complicated by serious organ failures or abnormalities in the patient's blood or metabolism.

Usually malaria is transmitted by the bite of an infected *Anopheles* mosquito, but cases of transfusion-transmitted malaria (TTM) have been recorded. TTM can be acquired through blood components such as red cell concentrates, platelets, leucocytes, plasma and frozen red blood cells. TTM is rare in countries in which malaria is not endemic. Nevertheless, this disease has an impact on the resources of blood banks because over the last few years more and more prospective donors have been coming from malaria endemic areas either as tourists, immigrants or workers. Moreover, donated blood may be imported from countries where Malaria is endemic. Furthermore, the disease is currently re-emerging in many areas where it had been eradicated in the past and, consequently, the number of subjects coming from malaria areas has risen. It should also be borne in mind that climate change has recently altered the distribution of mosquitos and is likely to allow malaria to spread into new countries.

In many countries, whole blood donation is deferred for several years in potential donors who lived the first 5 years of their life in malaria endemic areas, and for several months in potential donors returning from an endemic area and those who have been infected previously.

This period may, however, be reduced if an immunological or molecular genomic test is negative prior to a blood donation, increasing the pool of blood donors. Therefore, there is significant demand for diagnostically useful immunoassays.

A major obstacle for establishing of reliable immunoassays has been the lack of soluble antigens. Even the proteolytically obtained fragments of merozoite surface protein (MSP) are large proteins and extremely difficult to obtain in large quantities of sufficient biological activity using recombinant methods as would be required for establishing a routine assay.

The state of the art describes a range of immunoassays for detecting a malaria infection in blood samples. Meyerhof, A. S. et al (2010) Clin. and Vacc. Immunol., page 1631-1638, and WO2009/111599 disclose ELISA, IFA and bead-based immunoassay for the detection of antibodies to 19 kDa carboxyterminal regions of the merozoite surface proteins 1 (MSP-1) from *P*. *falciparum, P. vivax, P. ovale* and *P*. *malariae.* Using an assay based on beads coated with all four antigens the authors reported an IgG detection sensitivity of 100%. However, the assay appears to have limited specificity, since samples from healthy blood donors gave positive results. p33 is disclosed, but not as an antigen for a diagnostic assay.

Suitable assays have been described in the state of the art and already yield reasonably good diagnostic reliabilities. However, owing to the large number of blood donors and samples to be test and the considerable shortage of donated blood, any further optimization towards 100% sensitivity and specificity should be considered a valuable contribution to the art.

WO2011/06237 discloses a vaccine to protect against malaria comprising a p19 region from *P. falciparum* MSP-1 in combination with selected segments of p33, which have immunogenic properties in a vaccine. The document does not disclose that such a mixture may be applied to a diagnostic immunoassay, let alone an increased specificity in the sense that false negative results may be avoided.

Lucchi *et al.* (2008) disclose different antibody responses to the merozoite surface protein-I complex in cerebral malaria patients in India. Patients suffering from cerebral malaria are in a coma, though, and therefore unlikely to give blood. In addition, there is no pointer to p33, let alone that it may be used to increase the sensitivity of a test based on p19 (Lucchi et al. (2008) Antibody responses to the merozoite surface protein-I complex in cerebral malaria patients in India, Malaria Journal, 7:121).

Qian *et al.* (2012) discloses aggregates and conjugates of p42, which necessarily include a p19 fragment covalently attached to the corresponding p33 fragment. The p19 and p33 fragments (forming the p42 fragment) in the aggregates and conjugates are from the same strain (Plasmodium falciparum) (Qian et al. (2012) Immunogenicity of Self-Associated Aggregates and Chemically Cross-Linked Conjugates of the 42 kDa Plasmodium falciparum Merozoite Surface Protein-1. PLOS ONE 7(6): e36996. doi:l0.1371/journal.pone.0036996) .

Muerhoff *et al.* (2010) discloses ELISA and EIA assays using MSP-1 ₁₉ from *P*. *falciparum, P. vivax, P. ovale* and *P*. *malariae* immobilized on polystyrene beads as capture antigens. The assay is used to screen blood from potential donors. It explicitly mentions the possibility to combine antigens from different strains in a single immunoassay (by immobilizing a mix of antigens in the same bead) for improving the efficacy of the lest, however, it is silent about p33 (Muerhoff et al. (2010) Detection of Plasmodium falciparum, P. vivax, P. ovale, and P. malariae Merozoite Surface Protein 1-p19 Antibodies in Human Malaria Patients and Experimentally Infected Nonhuman Primates. CLINICAL AND VACCINE IMMUNOLOGY, Oct. 2010, p. 1631-1638).

Priest *et al.* (2018) discloses a multiplexed serological immunoassay for detecting malaria antibodies using MSP-1 ₁₉ from P. *falciparum, P. vivax, P. ovale and P. malariae* covalently immobilized on magnetic beads as capture antigens. It also discloses a method for determining the capacity of the carrier to bind the corresponding antibody by monitoring antibody binding at different dilutions and the use of these beads for purifying antibodies. This disclosure. however, is also silent about p33. Furthermore, antigen from different strains is immobilized on different beads (Priest et al. (2018) Specificity of the IgG antibody response to Plasmodium falciparum, Plasmodium vivax, Plasmodium malariae, and Plasmodium ovale MSP1 19 subunit proteins in multiplexed serologic assays. Malar J 17:417).

EP 2393824 discloses nucleic acid sequences and amino acid sequences encoded thereby, derived from the Merozoite Surface Protein (MSPI) gene of the Plasmodium species *P*. *malariae and P. ovale.* Such genes and proteins have many beneficial diagnostic as well as therapeutic uses.

US 2009/0226445 A1 discloses a methodology for determining the presence of antibodies against MSP-1 ₁₉ from *P*. *falciparum, P. vivax, P. ovale* and *P*. *malariae* in a sample using either a blend of beads coated with antigens from a single species, or beads coated with antigens from all four species. It is silent about p33.

US 2009/226445 discloses a method that involves contacting sample with a mixture of p19, p33 and p42 antigens from different strains. The "fused" polypeptides (p42) comprise p19 and p33 fragments from the same strain.

Pusic *et al.* (2011) discloses ELISA assays for detecting malaria antibodies using MSP-1 42 from P. falciparum covalently attached to polymer-coated quantum dots as capture antigen (see p. 8898-8900). In analogy to 01, the "fused" polypeptides (p42) comprise p19 and p33 fragments from the same strain (Pusic et al. (2011) Blood stage merozoite surface protein conjugated to nanoparticles induce potent parasite inhibitory antibodies. Vaccine 29 (2011) 8898-8908).

Fonseca *et al.* (2016) discloses a polypeptide comprising the P. *vivax* p19 elongated with a fragment of the *P*. *vivax* p33 and fused in tandem to several promiscuous T-cell epitopes. This modular chimera is used as a malaria vaccine. The "fused" chimera polypeptide comprises p19 and p33 fragments from the same strain (Fonseca et al. (2016) A chimeric protein-based malaria vaccine candidate induces robust T cell responses against Plasmodium vivax MSP119. SCIENTIFIC REPORTS (2016) 6:34527).

Kaira *et al.* (2016) discloses a fused polypeptide chimera comprising three antigenic regions of P. falciparum and is silent about p33 (Kaira et al. (2016) Antigenicity of a Bacterially Expressed Triple Chimeric Antigen of Plasmodium falciparum AARP, MSP-311 and MSP-1 19: PfAMSP-Fu35. PLOS ONE October 31, 2016 DOI:10.1371/journal.pone.0165720).

Therefore, the problem underlying the present invention is to provide an immunoassay for detecting antibodies to *Plasmodium* proteins that yields more reliable results than state of the art assays, particularly in terms of specificity, sensitivity and overall diagnostic reliability. It is desirable that such an immunoassay may be performed in a high-throughput manner for the purpose of confirming that donated blood is not infected. The assay format should be based on soluble proteins obtainable using recombinant methods for high-throughput use and standardization. It should be possible to identify healthy donors and diagnose TTM in patients who have received a blood transfusion as well as to screen donated fractionated blood.

The problem underlying the present invention is solved by the subject matter of the independent and dependent claims.

In a 1^{st} aspect, the problem underlying the present invention is solved by a polypeptide according to claim 1.

In a preferred embodiment, the polypeptide is immobilized, preferably on a diagnostically useful carrier.

In a preferred embodiment, the polypeptide is purified.

In a 2^{nd} aspect, the problem is solved by a diagnostically useful carrier coated with the polypeptide, preferably immobilized directly on the carrier, wherein the carrier is preferably selected from the group comprising a glass slide, preferably for microscopy, a biochip, a microtiter plate, a lateral flow device, a test strip, a membrane, preferably a line blot, a chromatography column and a bead, preferably a magnetic or fluorescent bead.

In a 3^{rd} aspect, the problem is solved by a method comprising the step detecting in a liquid four or five antibodies, preferably all from the group comprising
an antibody to p19 from *P*. *falciparum,*
an antibody to p19 from *P*. *vivax,*
an antibody to p19 from *P*. *ovale,*
an antibody to p19 from *P*. *malariae* and
an antibody to p19 from *P*. *knowlesi,*
comprising the step contacting the liquid with the polypeptide or carrier according to the present invention, wherein in addition an antibody to a p33 polypeptide from a *Plasmodium* strain is detected.

In a preferred embodiment, the antibody is detected using a method selected from the group comprising immunodiffusion techniques, immunoelectrophoretic techniques, light scattering immunoassays, agglutination techniques, labeled immunoassays such as those from the group comprising radiolabeled immunoassay, enzyme immunoassays, more preferably ELISA, chemiluminscence immunoassays, preferably electrochemiluminescence immunoassay, and immunofluorescence, preferably indirect immunofluorescence.

In a 4^{th} aspect, the problem is solved by a method comprising the step coating an analytically or diagnostically useful carrier with the polypeptide, wherein preferably the carrier is selected from the group comprising a glass slide, preferably for microscopy, a biochip, a microtiter plate, a lateral flow device, a test strip, a membrane, preferably a line blot, a chromatography column and a bead, preferably a magnetic or fluorescent bead.

In a 5^{th} aspect, the problem is solved by an *in-vitro* use of the polypeptide or diagnostically useful carrier for identifying blood from a non-infected blood donor.

In a preferred embodiment, one or more antibody is detected in a processed fraction of blood, preferably from a blood donor.

In a 6^{th} aspect, the problem is solved by a kit comprising the polypeptide or carrier, further comprising one or more reagents from the group comprising one or more than one calibrator, a washing buffer and a means for detecting an antibody, preferably a labeled secondary antibody.

In a 7^{th} aspect, the problem is solved by a use of the polypeptide for the manufacture of an analytical device, or for the manufacture of a composition, kit or reagent for detecting a Plasmodium infection.

In an 8^{th} aspect, the problem is solved by a method for determining the ability, preferably capacity of a carrier according to claim 4 to bind two or more antibodies to *Plasmodium* p19 proteins, comprising the step contacting the diagnostically useful carrier with one or more solutions comprising known concentrations of the two or more antibodies.

The present invention is based on the inventors' surprising finding that the use of a fusion polypeptide comprising four or five *Plasmodium* p19 polypeptides enhances the diagnostic reliability, particularly sensitivity of an immunoassay for the detection of *Plasmodium.* In particular, the detection signal is surprisingly enhanced and easier to distinguish from the background. Without meaning to be bound, it is theorized that the spatial proximity of the antigens has the effect that various antibodies to a range of epitopes are bound close to each other and that this leads to a higher density of antibodies that together are more straightforward to distinguish from any non-specifically binding antibodies or from any background signal.

The present invention relates to the detection of an antibody to *Plasmodium* p19 polypeptides based on a polypeptide comprising four or more of them. In a preferred embodiment, the term "*Plasmodium* p19", as used herein, is a polypeptide comprising a sequence element comprising the sequence SEQ ID NO1, wherein a, b, c and d are each at least 1 and a is more preferably 2 to 10, most preferably 5, and b is more preferably 2 to 10, most preferably 5 to 6, and c is more preferably 5 to 15, most preferably 9 to 13, and d is preferably, or a variant thereof. Preferably said polypeptide comprises a fragment from the wild type polypeptide from which the p19 is naturally cleaved or a variant of said fragment, wherein the fragment has a length of no more than 500, preferably 400, 300, 250, 200, 150, 100 or 90 amino acids. For example, if the *Plasmodium* p19 is from *P*. *falciparum,* the fragment is from the MSP-1 from *P. falciparum.*

In a more preferred embodiment, the *Plasmodium* p19 is selected from the group comprising p19 from *P*. *falciparum* (SEQ ID NO2) and a variant thereof, P. *vivax* (SEQ ID NO3) and a variant thereof, *P. ovale* (SEQ ID NO4) and a variant thereof, *P. malariae* (SEQ ID NO5) and a variant thereof and P. *knowlesi* (SEQ ID NO6) and a variant thereof. In a more preferred embodiment, one of the at least four or five *Plasmodium* p19 polypeptides is from P. *falciparum.* In another more preferred embodiment, one of the at least four or five *Plasmodium* p19 polypeptides is from *P*. *vivax.* In another more preferred embodiment, one of the at least four or five *Plasmodium* p19 polypeptides is from *P*. *ovale.* In another more preferred embodiment, one of the at least four or five *Plasmodium* p19 polypeptides is from *P. malariae.* In another more preferred embodiment, one of the at least four or five *Plasmodium* p19 polypeptides is from *P*. *knowlesi.*

A *Plasmodium* p19 is fused to a C-terminal *Plasmodium* p33 sequence, which is fused to the *Plasmodium* p19, more preferably to its N-terminus, optionally directly, or may in the fusion polypeptide according to the present invention be used as a linker between the two or more *Plasmodium* p19 polypeptides. In a preferred embodiment, this C-terminal Plasmodium p33 sequence is a sequence comprising at least 3, 4, 5, 6, 7, 8, 9, 10 or 15 or 17 amino acids from the C-terminus of a *Plasmodium* p33, more preferably comprising the consensus motif SXLL (SEQ ID NO14), or a variant thereof. The C-terminal p33 polypeptide may be from the same *Plasmodium* strain as the p19 to which it is fused, preferably directly fused. It may be selected from the group comprising SEQ ID NO20 (*P*. *vivax*)*,* SEQ ID NO22 (*P*. *falciparum*)*,* SEQ ID NO24 (*P*. *ovale*)*,* SEQ ID NO26 (*P*. *malariae*) and SEQ ID NO28 (*P*. *knowlesi*) and a variant thereof. In a preferred embodiment, a *Plasmodium* p19 fused to a C-terminal *Plasmodium* p33 sequence is part of the polypeptide and is selected from the group comprising SEQ ID NO21 (*P*. *vivax*)*,* SEQ ID NO23 (*P*. *falciparum*)*,* SEQ ID NO25 (*P*. *ovale*)*,* SEQ ID NO27 (*P*. *malariae*) and SEQ ID NO29 (*P*. *knowlesi*) and a variant thereof.

Terms implying that two *Plasmodium* p19 polypeptides and/or additional polypeptides are comprised by a polypeptide or are fused mean that they are covalently linked in a molecule that comprises these polypeptides. While this may be a direct fusion polypeptide in the sense that the entirety of the polypeptides fused or the polypeptide comprising these polypeptides is expressed based on a nucleic acid under the control of a promotor, which nucleic acid encodes the entirety of the polypeptide, a posttranslational link between separately expressed polypeptides by chemical ligation or chemical crosslinking is another option. The person skilled in the art is familiar with various chemical reactions and reagents such as crosslinkers, for example NHS based esters, carbodiimides, maleimides, haloacetyls are commercially available, and they are disclosed in the state of the art, for example in US6184344 or US7597882.

The polypeptide according to the present invention is a fusion comprising four or five *Plasmodium* p19 polypeptides, for example the p19 from P. *vivax* and the p19 from P. *falciparum;* the p19 from P. *vivax* and the p19 from P. *falciparum* and the p19 from P. *falciparum* and the p19 from P. *ovale.* In addition, it comprises one or more *Plasmodium* p33 polypeptides. The four or five p19 polypeptides and the p33 polypeptide, optionally additional p19 and/or p33 polypeptides, may be fused directly to each other or may be fused via linkers which are preferably polypeptide sequences comprising a *Plasmodium* or non*-Plasmodium* or an artificial sequence. This sequence is preferably chosen such that it is reasonably resistant to cleavage by proteolytic cleavage in an expression cell. Additionally, the sequence should not compromise the solubility of the entire polypeptide and should not limit access of antibodies in an aqueous environment of the polypeptide to epitopes in the p19 and/or p33 polypeptides.

The term "*Plasmodium* p33", as used herein is a polypeptide comprising a sequence element comprising the sequence (SEQ ID NO7), wherein a and b are each at least 1, and a is more preferably 15 to 60, most preferably 21, 23, 29, 35 or 45, and b is more preferably 10 to 30, most preferably 16, 22 or 23, or a variant thereof. Preferably said polypeptide comprises a fragment from the wild type polypeptide from which the p19 is naturally cleaved or a variant of said fragment, wherein the fragment has a length of no more than 800, preferably 700, 600, 500, 450, 400, 350 or 320 amino acids. For example, if the *Plasmodium* p33 is from P. *falciparum,* the fragment is from the MSP-1 from *P*. *falciparum.*

In a preferred embodiment, the *Plasmodium* p33 is selected from or comprises all from the group comprising p33 from *P. falciparum* (SEQ ID NO8) and a variant thereof, *P*. *vivax* (SEQ ID NO9) and a variant thereof, *P*. *ovale* (SEQ ID NO10) and a variant thereof, *P. malariae* (SEQ ID NO11) and a variant thereof and *P*. *knowlesi* (SEQ ID NO12) and a variant thereof. In a more preferred embodiment, the *Plasmodium* p33 is from *P*. *vivax.* In a more preferred embodiment, the *Plasmodium* p33 is from *P*. *falciparum.* In a more preferred embodiment, the *Plasmodium* p33 is from *P*. *ovale.* In a more preferred embodiment, the *Plasmodium* p33 is from *P*. *malariae.* In a more preferred embodiment, the *Plasmodium* p33 is from P. *knowlesi.*

The consensus sequences cited enable the person skilled in the art to use any p19 or p33 proteins from any further *Plasmodium strains* or parts thereof such as the C-terminus of p33, which may be discovered in the future, or from similar organisms, which cause malaria or a similar disease or need to be detected to ensure the quality of donated blood for another reason.

The merozoite surface protein (MSP-1) is a large multiprotein complex exposed on the surface of the merozoites. During late schizogony, MSP-1 is proteolytically processed from its 190 kDa precursor into four major cleavage products: p83, p30, p38 and p42. During erythrocyte invasion, the p42 is further cleaved into p33 and p19, which is essential for invasion. The proteolytically processed MSP-1 appears to exist in association with the processed products of MSP-6 and MSP-7.

According to the present invention, a diagnostically useful carrier is provided which comprises the polypeptide according to the present invention. Said polypeptide, together with the insoluble carrier to which it is attached, may be separated from a reaction mixture, wherein it is contacted with a liquid, in a straightforward manner, for example by filtration, centrifugation or decanting. Said polypeptide may be immobilized in a reversible or irreversible manner. For example, the immobilization is reversible if the polypeptide interacts with the carrier via ionic interactions which may be masked by addition of a high concentration of salt or if the polypeptide is bound via a cleavable covalent bond. By contrast, the immobilization is irreversible if the polypeptide is tethered to the carrier via a covalent bond that cannot be cleaved in aqueous solution. The polypeptide may be indirectly immobilized, for example by immobilizing an antibody or other entity having affinity to the polypeptide, followed by addition of the polypeptide and formation of a polypeptide-antibody complex. The antibody captured may be detected using a labeled secondary antibody or a labeled means for specifically capturing the antibody to be detected.

Throughout this application, the term "to capture specifically", also in variations such as "specifically capturing" or the like, as used herein, means that the binding reaction between the means and the antibody is stronger than a binding reaction characterized by a dissociation constant of 1 × 10⁻⁵ M, more preferably 1 × 10⁻⁷ M, more preferably 1 × 10⁻⁸ M, more preferably 1 × 10⁻⁹ M, more preferably 1 × 10⁻¹⁰ M, more preferably 1 × 10⁻¹¹ M, more preferably 1 × 10⁻¹² M, as determined by surface plasmon resonance using Biacore equipment at 25 °C in PBS buffer at pH 7.

In another preferred embodiment, the carrier is a microtiter plate comprising at least 8 wells that may be used for ELISA. At least one of the wells is directly or indirectly coated, for example using a coating antibody coupled to the plate which binds to the polypeptide. At least 3, preferably 4, more preferably 5 calibrators are provided that comprise an antibody to the polypeptide at defined concentrations and may be used to set up a calibration curve for semi-quantitative analysis. A secondary antibody comprising an enzymatically active label may be provided.

In another preferred embodiment, the diagnostically useful carrier comprises a polypeptide for specifically capturing any antibody of the class of antibody to be detected, preferably a means for specifically capturing all class, preferably IgG, IgA or IgM, preferably IgM class antibodies in the liquid, wherein the diagnostically useful carrier may be provided in combination with the polypeptide.

If that is the case, the assay is carried out such that the liquid is contacted with the carrier under conditions allowing binding to the carrier of all the antibodies in the liquid of the class of antibodies comprising the antibody to be detected, more specifically IgG class, preferably IgG antibody, followed by washing, followed by specific detection of the antibody to be detected. For this purpose, the carrier may be contacted with a labeled polypeptide according to the present invention.

The polypeptide or secondary antibody or any other means for detecting a complex comprising the polypeptide and an antibody from a sample may comprise a detectable label, preferably from the group comprising an enzymatically active, fluorescent, radioactive or luminescent, preferably chemiluminescent label or spin label. Alternatively the polypeptide may be dissociated from the antibody following specific binding and washing to detect its presence or absence.

Alternatively, a purified and labeled antibody may be used to detect a non-labeled antigen bound by the antibody to be detected which had in turn be captured by the means for specifically capturing all antibodies from the Ig class of the antibody to be detected, preferably IgG class, a method often referred to as bridge assay and described in various formats in the state of the art, for example US2018209973 or US5296347.

A competitive assay format may be used, wherein the polypeptide forms a complex with an antibody binding to it, following displacement of either the polypeptide or the antibody from the complex, as may be detected using a label which is attached to the displaced or displacing the polypeptide or antibody.

According to the present invention, a diagnostically useful carrier, preferably a bead, comprising the polypeptide is provided.

Various beads for numerous applications are commercially available, mainly based on carbohydrate, for example sepharose or agarose, or plastic. They contain active or activatable chemical groups such as carboxyl group, which can be utilized for the immobilization of reagents such as the polypeptide. Preferably, the beads are beads having an average diameter of from 0.2 µm to 5 mm, from 0.5 µm to 1 mm, from 0.75 µm to 100 µm or from 1 µm to 10 µm. The beads can be coated with the polypeptide directly or via affinity ligands, for example biotin or glutathione. Preferably, the bead is provided in the form of an aqueous suspension having a bead content of from 10 to 90%, preferably from 20 to 80%, preferably from 30 to 70%, more preferably from 40 to 60% (w/w).

In a particularly preferred embodiment, the beads are paramagnetic beads, which can be easily concentrated on a surface with the aid of a magnet. For this purpose, commercial paramagnetic beads usually contain a paramagnetic mineral, for example iron oxide. A multiplicity of suitable paramagnetic beads is commercially available.

The liquid used is any aqueous liquid that may comprise antibodies to be detected, preferably derived from blood. The liquid may preferably be a sample comprising antibodies from a subject. In a preferred embodiment, the sample is selected from the group comprising whole blood, serum, plasma, saliva, processed donated blood, preferably fractionated donated blood, more preferably fractions enriched in platelets or plasma or erythrocytes. Processed donated blood may comprise chemicals that stabilize it, for example by preventing clogging, preferably from the group comprising citrate, phosphate and dextrose.

However, the teachings of the present invention may not only be carried out using polypeptides having the exact sequences referred to in this application explicitly, for example by function, name, sequence or accession number, or implicitly, but also using variants of such polypeptides.

The term "variant", as used herein, may refer to at least one fragment of the full length sequence referred to, more specifically one or more amino acid or nucleic acid sequence which is, relative to the full-length sequence, truncated at one or both termini by one or more amino acids. Such a fragment comprises or encodes for a peptide having at least 10, 15, 25, 50, 75, 100, 150, 200 or 250 successive amino acids of the original sequence or a variant thereof. The total length of the variant may be at 25, 30, 40, 50, 60, 70, 80, 90, 100, 150, 200 or 250 or more amino acids.

The term "variant" relates not only to at least one fragment, but also a polypeptide or a fragment thereof comprising amino acid sequences, preferably a fragment comprising at least 25, more preferably 50, more preferably 200 successive amino acids, that are at least 40, 50, 60, 70, 75, 80, 85, 90, 92, 94, 95, 96, 97, 98 or 99 % identical to the reference amino acid sequence referred to or the fragment thereof, wherein amino acids other than those essential for the biological activity, for example the ability to bind specifically to an antibody of interest, or the fold or structure of the polypeptide are deleted or substituted and/or one or more such essential amino acids are replaced in a conservative manner and/or amino acids are added or deleted such that the biological activity of the polypeptide is at least partially preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007): Clustal W and Clustal X version 2.0. Bioinformatics, 23, 2947-2948) is used applying default settings.

Variants may, in addition, comprise chemical modifications, for example labels such as isotopic labels or detectable labels or covalent modifications such as glycosylation, phosphorylation, acetylation, decarboxylation, citrullination, hydroxylation and the like. The person skilled in the art is familiar with methods for the modification of polypeptides. Moreover, variants may also be generated by way of fusion with other known polypeptides or variants thereof, for example artificial linkers, preferably not derived from a *Plasmodium* polypeptide, affinity tags, other antigens and the like.

The variant of the polypeptide has biological activity. In a preferred embodiment such biological activity is the ability to bind to the respective antibody. In a preferred embodiment it comprises an epitope having the ability to bind to the respective antibody, preferably from a sample from a patient suffering from Malaria, wherein more preferably the epitope comprises a sequence comprising at least 5, 6, 7 or 8 amino acid residues. More specifically, a variant of SEQ X has the ability to bind specifically to or specifically capture an antibody binding to SEQ X from a sample from a patient infected with *Plasmodium,* wherein X is preferably selected from the group comprising SEQ ID NO1, SEQ ID NO2, SEQ ID NO3, SEQ ID NO4, SEQ ID NO5, SEQ ID NO6, SEQ ID NO7, SEQ ID NO8, SEQ ID NO9, SEQ ID NO10, SEQ ID NO11, SEQ ID NO12, SEQ ID NO13, SEQ ID NO14, SEQ ID NO15, SEQ ID NO16, SEQ ID NO17, SEQ ID NO18, SEQ ID NO19, SEQ ID NO20, SEQ ID NO21, SEQ ID NO22, SEQ ID NO23, SEQ ID NO24, SEQ ID NO25, SEQ ID NO26, SEQ ID NO27, SEQ ID NO28 and SEQ ID NO29.

The person skilled in the art is capable of designing variants having biological activity by starting from the respective sequence X, if a variant of X is required, introduce modifications such as point mutations, truncations and the like and subsequently confirming that the variant still has biological activity by testing whether said variant binds to an antibody from such a sample. In a preferred embodiment, an ELISA assay based on variant polypeptide coated in a microplate, preferably as described in the examples, is used to test whether the variant has such activity.

The polypeptide may be provided in any form and at any degree of purification, from tissues, fruits or cells comprising said polypeptide in an endogenous form, more preferably cells overexpressing the polypeptide, crude or enriched lysates of such cells, to purified and/or isolated polypeptide which may be essentially pure. In a preferred embodiment, the polypeptide is a native polypeptide, wherein the term "native polypeptide", as used herein, refers to a folded polypeptide, more preferably to a folded polypeptide purified from cells, more preferably from mammalian cells. If a native polypeptide is used, it is preferably enriched compared to its natural state.

According to the present invention, the polypeptide may be a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification). In another preferred embodiment, the polypeptide is an isolated polypeptide, wherein the term "isolated" means that the polypeptide has been enriched compared to its state upon production using a biotechnological or synthetic approach and is preferably pure, *i.e.* at least 60, 70, 80, 90, 95 or 99 percent of the polypeptide in the respective liquid consists of said polypeptide as judged by SDS polyacrylamide gel electrophoresis followed by Coomassie blue staining and visual inspection. Preferably any polypeptide on a carrier used as a means to capture an antibody is pure.

Any antibody may be detected in a liquid, preferably a sample from a subject. The is an organism producing antibodies, preferably IgM, IgA or IgG class antibodies, more preferably IgG class antibodies, more preferably a mammal, most preferably a human.

The inventive teachings provide a kit, preferably for detecting *Plasmodium* infection or diagnosing Malaria, more preferably for diagnosing TTM. Such a kit is a container that comprises specific reagents required to practice the inventive method, in particular the diagnostically useful carrier according to the present invention, optionally in addition to one or more solutions or reagents required to practice the inventive method, preferably selected from or all from the group comprising sample dilution buffer, washing buffer and buffer comprising a means for detecting any specifically captured antibody, such as a secondary antibody and optionally a means for detecting the latter. Furthermore, it may comprise instructions detailing how to use the kit. It may comprise the diagnostically useful carrier for contacting the inventive polypeptide with a bodily fluid sample from a subject, preferably a human subject, for example a line blot. Furthermore, the kit may comprise a positive control, for example a recombinant antibody known to bind to one or more from the group comprising SEQ ID NO1, SEQ ID NO2, SEQ ID NO3, SEQ ID NO4, SEQ ID NO5, SEQ ID NO6, SEQ ID NO7, SEQ ID NO8, SEQ ID NO9 and SEQ ID NO10, and a negative control, for example a protein having no detectable affinity to a *Plasmodium* p19 or p33 such as bovine serum albumin. Finally, such a kit may comprise one or more standard solutions comprising an antibody binding to one or more from SEQ ID NO1, SEQ ID NO2, SEQ ID NO3, SEQ ID NO4, SEQ ID NO5, SEQ ID NO6, SEQ ID NO7, SEQ ID NO8, SEQ ID NO9 and SEQ ID NO10 for preparing a calibration curve, wherein the absolute or relative concentration of the antibody in each standard solution is preferably known. The kit may comprise a means or reagent for detecting a captured antibody. The kit may comprise the polypeptide according to the present invention or a purified polypeptide comprising one sequence from the group comprising SEQ ID NO1, SEQ ID NO2, SEQ ID NO3, SEQ ID NO4, SEQ ID NO5, SEQ ID NO6, SEQ ID NO7, SEQ ID NO8, SEQ ID NO9 and SEQ ID NO10, which polypeptide may optionally be labeled. The kit may also comprise a washing solution. The kit may comprise a suitable water-tight vessel for contacting the diagnostically useful carrier with the sample in the presence of other liquids such as a reaction buffer. For example, a line blot may be provided in or in combination with an incubation tray, or a microtiter plate may be provided. Suitable vessels are described in the state of the art, for example EP3025780 or EP3025779).

According to the present invention, a means or reagent for detecting a captured antibody may be provided or used to detect a captured antibody to a *Plasmodium* p19 and/or to detect an antibody to a *Plasmodium* p33. This means or reagent may be a secondary antibody binding to the constant region of an antibody of the class of interest. For example, if a captured human IgG class antibody is to be detected, an antibody binding specifically to the constant region of human IgG class antibodies may be used. The means or reagent may be directly or indirectly labeled. It binds specifically, which preferably means that the binding reaction between the means and the captured antibody is stronger than a binding reaction characterized by a dissociation constant of 1 × 10⁻⁵ M, more preferably 1 × 10⁻⁷ M, more preferably 1 × 10⁻⁸ M, more preferably 1 × 10⁻⁹ M, more preferably 1 × 10⁻¹⁰ M, more preferably 1 × 10⁻¹¹ M, more preferably 1 × 10⁻¹² M, as determined by surface plasmon resonance using Biacore equipment at 25 °C in PBS buffer at pH 7.

The products and methods according to the present invention may be used for detecting a *Plasmodium* infection or contamination in a liquid such as donated blood *in vitro.* The donated blood is preferably from a donor whose identity is unknown. The blood is likely or more likely to be contaminated with *Plasmodium* if an antibody to at least one *Plasmodium* p19 or at least one *Plasmodium* p33 is detected.

They may also be used for diagnosing a disease which is a *Plasmodium* infection, preferably Malaria, more preferably TTM in a subject. In a preferred embodiment, the term "diagnosis", as used herein, refers to any kind of procedure aiming to obtain information instrumental in the assessment whether a patient suffers or is likely or more likely than the average or a comparative subject, the latter preferably having similar symptoms, to suffer from certain a disease or disorder in the past, at the time of the diagnosis or in the future, to find out how the disease is progressing or is likely to progress in the future or to evaluate the responsiveness of a patient with regard to a treatment. In other words, the term "diagnosis" comprises not only diagnosing, but also prognosticating and/or monitoring the course of a disease or disorder. The subject is likely or more likely to suffer from the disease if an antibody to at least one *Plasmodium* p19 or at least one *Plasmodium* p33 as shown by the detection of an antibody to the polypeptide according to the invention is detected in his liquid.

Therefore, the term "diagnosis" does preferably not imply that the diagnostic methods or agents according to the present invention will be definitive and sufficient to finalize the diagnosis on the basis of a single test, let alone parameter, but may refer to a contribution to what is referred to as a "differential diagnosis", *i.e.* a systematic diagnostic procedure considering the likelihood of a range of possible conditions on the basis of a range of diagnostic parameters. In a preferred embodiment, the term "diagnosis" means that the method or product or use may be used for aiding in the diagnosis of a disease or identifying a subject a risk of suffering from a disease. The term "diagnosis" may also refer to a method or agent used to choose the most promising treatment regime for a patient. In other words, the method or agent may relate to selecting a treatment regimen for a subject.

The present invention relates to a method comprising the step detecting in a liquid from a subject the presence or absence of an antibody to a *Plasmodium* p19 and/or a *Plasmodium* p33. Such a method may comprise the steps a) providing a liquid, preferably a sample from donated processed and/or fractionated blood or sample from a subject, b) contacting the liquid with the polypeptide according to the present invention under conditions compatible with the formation of a complex comprising the diagnostically useful carrier and the antibody, more specifically the polypeptide and the antibody, c) isolating any said complex, for example by removing the liquid, d) optionally washing said complex, and e) detecting said complex, optionally after contacting with a *Plasmodium* p19 and/or *Plasmodium* p33, which may be labeled in the case of a competitive assay format. The method is preferably an *in vitro* method.

The detection of the antibody or complex for the prognosis, diagnosis, methods or kit according to the present invention comprises the use of a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemiluminscence immunoassays and immunofluorescence techniques. In a preferred embodiment, the complex is detected using a method selected from the group comprising immunodiffusion techniques, immunoelectrophoretic techniques, light scattering immunoassays, agglutination techniques, labeled immunoassays from the group comprising radiolabeled immunoassays, chemiluminscence immunoassays and immunofluorescence techniques. The person skilled in the art is familiar with these methods, which are also described in the state of the art, for example in Zane, H. D. (2001): Immunology - Theoretical & Practical Concepts in Laboratory Medicine, W. B. Saunders Company, in particular in Chapter 14.

In another preferred embodiment, the prognosis, diagnosis, methods or kit in line with the inventive teachings contemplate the use of indirect immunofluorescence. The person skilled in the art is familiar with such techniques, which are described in the state of the art (US4647543; Voigt, J., Krause, C., Rohwäder, E, Saschenbrecker, S., Hahn, M., Danckwardt, M., Feirer, C., Ens, K, Fechner, K, Barth, E, Martinetz, T., and Stöcker, W. (2012), Automated Indirect Immunofluorescence Evaluation of Antinuclear Autoantibodies on HEp-2 Cells," Clinical and Developmental Immunology, vol. 2012, doi:10.1155/2012/65105; Bonilla, E., Francis, L., Allam, F., et al., Immuno-fluorescence microscopy is superior to fluorescent beads for detection of antinuclear antibody reactivity in systemic lupus erythematosus patients, Clinical Immunology, vol. 124, no. 1, pp. 18-21, 2007). Suitable reagents, devices and software packages are commercially available, for example from EUROIMMUN, Lübeck, Germany.

In many cases detecting, preferably meaning detecting the absence or presence of an antibody, optionally meaning determining whether the concentration of the antibody is beyond a certain threshold preferably as set by measurement using ELISA, in the liquid, is sufficient for the diagnosis. If the antibody can be detected, this will be information instrumental for the clinician's diagnosis and indicates an increased likelihood that the patient suffers from a disease. In a preferred embodiment, the relative concentration of the antibody in the serum, compared to the level that may be found in the average healthy subject, may be determined. In a preferred embodiment, the term "detecting the presence", as used herein, means that it is sufficient to check whether a signal sufficiently beyond any background level may be detected using a suitable complex detection method that indicates that the antibody of interest is present or more antibody of interest is present than would be in a healthy subject.

In a preferred embodiment, the absence or presence of two or more antibodies to a *Plasmodium* p19 and optionally one or more antibodies to a *Plasmodium* p33 is preferably detected simultaneously, *i.e.* at the same time.

In a preferred embodiment, the absence or presence of two or more antibodies to a *Plasmodium* p19 and optionally one or more antibodies to a *Plasmodium* p33 is detected in spatially separate reactions, more preferably in different reaction mixtures in separate vessels.

According to the present invention, four or five antibodies to a *Plasmodium* p19 and an antibody to a *Plasmodium* p33 and optionally additional antibodies are detected. In a preferred embodiment, this means that it is detected whether at least four or five of these antibodies are present, without distinguishing to which of the antigens the antibodies bind or whether only five or more than five antibodies are present. In any event, it is important to ensure that antibodies who bind to an antigen other than a *Plasmodium* p19 and *Plasmodium* p33 are not mistakenly taken as antibody binding to a *Plasmodium* p19 and *Plasmodium* p33 to avoid false positive results.

In a preferred embodiment, the invention is used to check on an analytical device. In a preferred embodiment, the term "analytical device" refers to a device made for non-diagnostic purposes, in particular for detecting at least one antibody to a *Plasmodium* p19 and at least one antibody to a *Plasmodium* p33 for screening donated blood. In other words, the information gathered is used to check the quality of donated blood, with the identity of the donor unknown, rather than diagnosing the condition of a known patient, with assignment of a diagnostic result to a specific person.

The invention provides a use of the polypeptide according to the present invention for the manufacture of a kit, reagent or composition for the diagnosis of a disease or for screening donated blood for infection or contamination with *Plasmodium,* wherein preferably a diagnostic or analytical assay with an increased sensitivity is provided. Such manufacture may relate to a method comprising the step immobilizing on a diagnostically useful carrier or analytical device the polypeptide according to the present invention.

### Sequences:

Reference is made to a range of novel polypeptides, more specifically
**SEQ ID NO1 (*Plasmodium* p19 consensus motif)** 
HXC(Xₐ)P(E/D)NXXCXRXXDXXEXXXCLLXXKXXXXXCV(X_{b})CXXNNGGCXXXAXC(X_{c})CXC(
X_{d})F(D/E)GXFCX,
 wherein each of a, b, c and d is at least 1.
**SEQ ID NO2 (*P. falciparum* p19)** 
NISQHQCVKKQCPENSGCFRHLDEREECKCLLNYKQEGDKCVENPNPTCNENNGGCDAD
AKCTEEDSGSNGKKITCECTKPDSYPLFDGIFCS
**SEQ ID NO3 (*P. vivax* p19)** 
TMSSEHTCIDTNVPDNAACYRYLDGTEEWRCLLTFKEEGGKCVPASNVTCKDNNGGCAPE
AECKMTDSNKIVCKCTKEGSEPLFEGVFCS
**SEQ ID NO4 (*P. ovale* p19)** 
SMGSKHKCIDITYPDNAGCYRFSDGREEWRCLLNFKKVGETCVPNNNPTCAENNGGCDPT
ADCAESENNKITCTCTGQNESFFEGVFCG
**SEQ ID NO5 (*P. malariae* p19)** 
NISAKHACTETKYPENAGCYRYEDGKEVWRCLLNYKLVDGGCVEDEEPSCQVNNGGCAPE
ANCTKGDDNKIVCACNAPYSEPIFEGVFCG
**SEQ ID NOG (*P. knowlesi* p19)** 
NMSSAHKCIDTNVPENAACYRYLDGTEEWRCLLGFKEVGGKCVPASITCEENNGGCAPEA
ECTMDDKKEVECKCTKEGFEPLFEGVFCS
**SEQ ID NO7 (*P*. p33 consensus motif)** 
YXVXYXKPLAGXYXXXXKQXXXXXXXFXXNXXXXLXXRXKKRXYFLXVLXSXLXXXKXXXSX
XYXIXDXXKLLXXXXKXXLXXSYXYIXXXXXXDXXXAXXGXXYYXKXXXXYKXXL(Xa)KXXPFL
 
XXXXXXYXXLXXXXXXXXXXLKXXXXXCXXEKXXXXXXXXKLXXXXXXDXXXXXXK(Xb)LXX
XXXXXXXXXXXXXXSXL,
 wherein each of a and b is at least 1
**SEQ ID NO8 (*P. falciparum p33*)** 
AISVTMDNILSGFENEYDVIYLKPLAGVYRSLKKQIEKNIFTFNLNLNDILNSRLKKRKYFLDVL
ESDLMQFKHISSNEYIIEDSFKLLNSEQKNTLLKSYKYIKESVENDIKFAQEGISYYEKVLAKYK
DDLESIKKVIKEEKEKFPSSPPTTPPSPAKTDEQKKESKFLPFLTNIETLYNNLVNKIDDYLINL
KAKINDCNVEKDEAHVKITKLSDLKAIDDKIDLFKNPYDFEAIKKLINDDTKKDMLGKLLSTGLV
QNFPNTIISKLIEGKFQDML
**SEQ ID NO9 (*P. vivax* p33)** 
DQVTTGEAESEAPEILVPAGISDYDVVYLKPLAGMYKTIKKQLENHVNAFNTNITDMLDSRLK
KRNYFLEVLNSDLNPFKYSSSGEYIIKDPYKLLDLEKKKKLIGSYKYIGASIDMDLATANDGVT
YYNKMGELYKTHLDGVKTEIKKVEDDIKKQDEELKKLGNVNSQDSKKNEFIAKKAELEKYLP
FLNSLQKEYESLVSKVNTYTDNLKKVINNCQLEKKEAEITVKKLQDYNKMDEKLEEYKKSEK
KNEVKSSGLLEKLMKSKLIKENESKEILSQLLNVQTQLL
**SEQ ID NO10 (*P. ovale* p33)** 
AQDKDDNILNEITNEYEVVYVKPLAGVYKTLKKQLEAHVTAFHSNVTNMLESRLKKRNYFLE
VLNSDLTQYKHATSDNYVIRDAYKLLDFEKKKKLLSSYKYIKDSVEKDVEIATDGIDYYEKMA
ALYKTYLESVNAQVDAIDKTGDDATKATNKKFLPFLASINAMYETLLEKVNTYNSQLKSSLNS
CQLEKIRVGIVVDKLNDYVMFDEKLEELKSSKEKDLTKLYKDIDTSNIINKLKRSGFVDTDESK
KLLSELLDVDSAQLL
**SEQ ID NO11 (*P. malariae* p33)** 
AETKQDDNILKPITNEYEVLYIKPLAGVYRVLRKQIGDQIDAFNSNLTNALDTRKKKRTYFLDV
LNSDLIQFKHATSDSYIIKDPYKLLDVDKKAKLIGSYKYIVSAIEKDITSAENGVEYYDKMTKLY
KTQLEAVKSAIAEAQKEGDKKTENEKYIPFLTNMQTLYENLLNKINGNIINLKTLITNCNLEKDA
VNITISKLTEYSKFDEKIEMFKNSKNEKDIASSGILDILKQKGLVNKNESTKIISELLGVDSNALL
**SEQ ID NO12 (*P. knowlesi* p33)** 
AENAQPENIVPEGINEYEVVYIKPLAGMYKSIKKQLENHVAAFNTNITDMLDSRLKKRNYFLD
VLDSELNPFKYSSSGEYIIKDPYKLLDLEQKKKLLGSYQYIGASVDKDLITAKDGMEYYNKMG
ELYKQHLEAVNAQIKEIEASVPGEQSQLNAQKEELKKYLPFLNSIQKEYESLVNMAHTYKENL
KKFINNCQIEKKETEIIVKKLEDYTKIDENLEIYKKSKKESDVRSSGLLEKLKNSKLINEEESKK
VLSQLLNVQTQML
**SEQ ID NO13 (His-tagged p19 from *P. falciparum*)**
   **>P6527_His-MSP-1-p19_P.falciparum_B140110RD** 
MSHHHHHHHHSMNLAKTVLSNLLDGNLQGMLNISQHQCVKKQCPENSGCFRHLDEREEC
KCLLNYKQEGDKCVENPNPTCNENNGGCDADAKCTEEDSGSNGKKITCECTKPDSYPLFD
GIFCS
**SEQ ID NO14 (p33 C-terminal consensus motif)**
   **>MSP p33 C-terminal consensus motif**
   SXLL
**SEQ ID NO15 (His-tagged p19 from *P. vivax*)**
   **>P6526_His-MSP-1-p19_P.vivax_B140110RE** 
MSHHHHHHHHSMENESKEILSQLLNVQTQLLTMSSEHTCIDTNVPDNAACYRYLDGTEEW
RCLLTFKEEGGKCVPASNVTCKDNNGGCAPEAECKMTDSNKIVCKCTKEGSEPLFEGVFCS
**SEQ ID NO16 (His-tagged p33 from *P. vivax*)**
   **>P16463_His-MSP-1-p33_P.vivax_B180806RA** 
MSHHHHHHHHSMSDQVTTGEAESEAPEILVPAGISDYDVVYLKPLAGMYKTIKKQLENHVN
AFNTNITDMLDSRLKKRNYFLEVLNSDLNPFKYSSSGEYIIKDPYKLLDLEKKKKLIGSYKYIG
ASIDMDLATANDGVTYYNKMGELYKTHLDGVKTEIKKVEDDIKKQDEELKKLGNVNSQDSKK
NEFIAKKAELEKYLPFLNSLQKEYESLVSKVNTYTDNLKKVINNCQLEKKEAEITVKKLQDYN
KMDEKLEEYKKSEKKNEVKSSGLLEKLMKSKLIK
**SEQ ID NO17 (His-tagged p19 from *P. ovale*)**
   **>P6528_His-MSP-1-p19_P.ovale_B140110RC** 
MSHHHHHHHHSMDESKKLLSELLDVDSAQLLSMGSKHKCIDITYPDNAGCYRFSDGREEW
RCLLNFKKVGETCVPNNNPTCAENNGGCDPTADCAESENNKITCTCTGQNESFFEGVFCG
**SEQ ID NO18 (His-tagged p19 from *P. malariae*)**
   **>P6529_His-MSP-1-p19_P.malariae_B140110RB** 
MSHHHHHHHHSMNESTKIISELLGVDSNALLNISAKHACTETKYPENAGCYRYEDGKEVWR
CLLNYKLVDGGCVEDEEPSCQVNNGGCAPEANCTKGDDNKIVCACNAPYSEPIFEGVFCG
**SEQ ID NO19 (His-tagged p19 from *P. knowlesi*)**
   **>P6530_His-MSP-1-p19_P.knowlesi_B140110RA** 
MSHHHHHHHHSMEEESKKVLSQLLNVQTQMLNMSSAHKCIDTNVPENAACYRYLDGTEE
WRCLLGFKEVGGKCVPASITCEENNGGCAPEAECTMDDKKEVECKCTKEGFEPLFEGVFC
S
**SEQ ID NO20 (delta-p33 from *P. vivax*)**
   **> MSP1[delta-p33]_P.vivax**
   NESKEILSQLLNVQTQLL
**SEQ ID NO 21 (delta-p33-p19 from *P. vivax*)**
   **> MSP1[delta-p33-p19]_P.vivax** 
NESKEILSQLLNVQTQLLTMSSEHTCIDTNVPDNAACYRYLDGTEEWRCLLTFKEEGGKCVP
ASNVTCKDNNGGCAPEAECKMTDSNKIVCKCTKEGSEPLFEGVFCS
**SEQ ID NO22 (delta-p33 from *P. falciparum*)**
   **> MSP1[delta-p33]_P.falciparum**
   NLAKTVLSNLLDGNLQGML
**SEQ ID NO23 (delta-p33-p19 from *P. falciparum*)**
   **> MSP1[delta-p33-p19]_P.falciparum** 
NLAKTVLSNLLDGNLQGMLNISQHQCVKKQCPENSGCFRHLDEREECKCLLNYKQEGDKC
VENPNPTCNENNGGCDADAKCTEEDSGSNGKKITCECTKPDSYPLFDGIFCS
**SEQ ID NO24 (delta-p33 from *P. ovale*)**
   **> MSP1[delta-p33]_P.falciparum**
   DESKKLLSELLDVDSAQLL
**SEQ ID NO25 (delta-p33-p19 from *P. ovale*)**
   **> MSP1[delta-p33-p19]_P.ovale** 
DESKKLLSELLDVDSAQLLSMGSKHKCIDITYPDNAGCYRFSDGREEWRCLLNFKKVGETC
VPNNNPTCAENNGGCDPTADCAESENNKITCTCTGQNESFFEGVFCG
**SEQ ID NO26 (delta-p33 from *P. malariae*)**
   **> MSP1[delta-p33]_P.malariae**
   NESTKIISELLGVDSNALL
**SEQ ID NO27 (delta-p33-p19 from *P. malariae*)**
   **> MSP1[delta-p33-p19]_P.malariae** 
NESTKIISELLGVDSNALLNISAKHACTETKYPENAGCYRYEDGKEVWRCLLNYKLVDGGCV
EDEEPSCQVNNGGCAPEANCTKGDDNKIVCACNAPYSEPIFEGVFCG
**SEQ ID NO28 (delta-p33 from *P. knowlesi*)**
   **> MSP1[delta-p33]_P.knowlesi**
   EEESKKVLSQLLNVQTQML
**SEQ ID NO29 (delta-p33-p19 from *P. knowlesi*)**
   **> MSP1[delta-p33-p19]_P.knowlesi** 
EEESKKVLSQLLNVQTQMLNMSSAHKCIDTNVPENAACYRYLDGTEEWRCLLGFKEVGGK
CVPASITCEENNGGCAPEAECTMDDKKEVECKCTKEGFEPLFEGVFCS
**SEQ ID NO30 ("His-MSP-multi-p19" - Purified fusion polypeptide according to the present invention, N-terminally His tagged, p33 from *P. vivax* - p19 from *P. vivax* - C-terminal *P. falciparum* p33 sequence - p19 from *P. falciparum* - C-terminal *P*. *ovale* p33 sequence - p19 from *P. ovale* - C-terminal P. malariae p33 sequence - p19 from P. malariae- C-terminal *P. knowlesi* p33 sequence - p19 from *P*. *knowlesi*)** 
MSHHHHHHHHSMSDQVTTGEAESEAPEILVPAGISDYDVVYLKPLAGMYKTIKKQLENHVN
AFNTNITDMLDSRLKKRNYFLEVLNSDLNPFKYSSSGEYIIKDPYKLLDLEKKKKLIGSYKYIG
ASIDMDLATANDGVTYYNKMGELYKTHLDGVKTEIKKVEDDIKKQDEELKKLGNVNSQDSKK
NEFIAKKAELEKYLPFLNSLQKEYESLVSKVNTYTDNLKKVINNCQLEKKEAEITVKKLQDYN
KMDEKLEEYKKSEKKNEVKSSGLLEKLMKSKLIKENESKEILSQLLNVQTQLLTMSSEHTCID
TNVPDNAACYRYLDGTEEWRCLLTFKEEGGKCVPASNVTCKDNNGGCAPEAECKMTDSN
KIVCKCTKEGSEPLFEGVFCSNLAKTVLSNLLDGNLQGMLNISQHQCVKKQCPENSGCFRH
LDEREECKCLLNYKQEGDKCVENPNPTCNENNGGCDADAKCTEEDSGSNGKKITCECTKP
DSYPLFDGIFCSDESKKLLSELLDVDSAQLLSMGSKHKCIDITYPDNAGCYRFSDGREEWRC
LLNFKKVGETCVPNNNPTCAENNGGCDPTADCAESENNKITCTCTGQNESFFEGVFCGNE
STKIISELLGVDSNALLNISAKHACTETKYPENAGCYRYEDGKEVWRCLLNYKLVDGGCVED
EEPSCQVNNGGCAPEANCTKGDDNKIVCACNAPYSEPIFEGVFCGEEESKKVLSQLLNVQT
QMLNMSSAHKCIDTNVPENAACYRYLDGTEEWRCLLGFKEVGGKCVPASITCEENNGGCA
PEAECTMDDKKEVECKCTKEGFEPLFEGVFCSTMEG
**SEQ ID NO31 ("His-MSP-multi-His_a" - Purified fusion polypeptide according to the present invention, N-terminally His tagged p33 from *P. vivax* - C-terminal *P. knowlesi* p33 sequence - p19 from *P. knowlesi -* p19 from *P. vivax -* C-terminal *P. falciparum* p33 sequence - p19 from *P. falciparum* - C-terminal *P. ovale* p33 sequence - p19 from *P. ovale* - C-terminal P. malariae p33 sequence - p19 from P. malariae)** 
MSHHHHHHHHSMSDQVTTGEAESEAPEILVPAGISDYDVVYLKPLAGMYKTIKKQLENHVN
AFNTNITDMLDSRLKKRNYFLEVLNSDLNPFKYSSSGEYIIKDPYKLLDLEKKKKLIGSYKYIG
ASIDMDLATANDGVTYYNKMGELYKTHLDGVKTEIKKVEDDIKKQDEELKKLGNVNSQDSKK
NEFIAKKAELEKYLPFLNSLQKEYESLVSKVNTYTDNLKKVINNCQLEKKEAEITVKKLQDYN
KMDEKLEEYKKSEKKNEVKSSGLLEKLMKSKLIKENESKEILSQLLNVQTQLLAMEEESKKVL
SQLLNVQTQMLNMSSAHKCIDTNVPENAACYRYLDGTEEWRCLLGFKEVGGKCVPASITCE
ENNGGCAPEAECTMDDKKEVECKCTKEGFEPLFEGVFCSTMSSEHTCIDTNVPDNAACYR
YLDGTEEWRCLLTFKEEGGKCVPASNVTCKDNNGGCAPEAECKMTDSNKIVCKCTKEGSE
PLFEGVFCSNLAKTVLSNLLDGNLQGMLNISQHQCVKKQCPENSGCFRHLDEREECKCLLN
YKQEGDKCVENPNPTCNENNGGCDADAKCTEEDSGSNGKKITCECTKPDSYPLFDGIFCS
DESKKLLSELLDVDSAQLLSMGSKHKCIDITYPDNAGCYRFSDGREEWRCLLNFKKVGETC
VPNNNPTCAENNGGCDPTADCAESENNKITCTCTGQNESFFEGVFCGNESTKIISELLGVDS
NALLNISAKHACTETKYPENAGCYRYEDGKEVWRCLLNYKLVDGGCVEDEEPSCQVNNGG
CAPEANCTKGDDNKIVCACNAPYSEPIFEGVFCG
**SEQ ID NO32 ("His-MSP-multi-His_b" - Purified fusion polypeptide according to the present invention, N-terminally His tagged p33 from *P. vivax* - C-terminal *P. malariae* p33 sequence - p19 from P. malariae - C-terminal *P. knowlesi* p33 sequence - p19 from *P. knowlesi* - p19 from *P. vivax -* C-terminal *P. falciparum* p33 sequence - p19 from *P. falciparum* - C-terminal *P. ovale* p33 sequence - p19 from *P. ovale*)** 
MSHHHHHHHHSMSDQVTTGEAESEAPEILVPAGISDYDVVYLKPLAGMYKTIKKQLENHVN
AFNTNITDMLDSRLKKRNYFLEVLNSDLNPFKYSSSGEYIIKDPYKLLDLEKKKKLIGSYKYIG
ASIDMDLATANDGVTYYNKMGELYKTHLDGVKTEIKKVEDDIKKQDEELKKLGNVNSQDSKK
NEFIAKKAELEKYLPFLNSLQKEYESLVSKVNTYTDNLKKVINNCQLEKKEAEITVKKLQDYN
KMDEKLEEYKKSEKKNEVKSSGLLEKLMKSKLIKENESKEILSQLLNVQTQLLAMNESTKIISE
LLGVDSNALLNISAKHACTETKYPENAGCYRYEDGKEVWRCLLNYKLVDGGCVEDEEPSC
QVNNGGCAPEANCTKGDDNKIVCACNAPYSEPIFEGVFCGEEESKKVLSQLLNVQTQMLN
MSSAHKCIDTNVPENAACYRYLDGTEEWRCLLGFKEVGGKCVPASITCEENNGGCAPEAE
CTMDDKKEVECKCTKEGFEPLFEGVFCSTMSSEHTCIDTNVPDNAACYRYLDGTEEWRCLL
TFKEEGGKCVPASNVTCKDNNGGCAPEAECKMTDSNKIVCKCTKEGSEPLFEGVFCSNLA
KTVLSNLLDGNLQGMLNISQHQCVKKQCPENSGCFRHLDEREECKCLLNYKQEGDKCVEN
 
PNPTCNENNGGCDADAKCTEEDSGSNGKKITCECTKPDSYPLFDGIFCSDESKKLLSELLDV
DSAQLLSMGSKHKCIDITYPDNAGCYRFSDGREEWRCLLNFKKVGETCVPNNNPTCAENN
GGCDPTADCAESENNKITCTCTGQNESFFEGVFCG
**SEQ ID NO33 ("His-MSP-multi-His_c" - Purified fusion polypeptide according to the present invention, N-terminally His tagged p33 from *P. vivax-* C-terminal *P. ovale* p33 sequence - p19 from *P. ovale* - C-terminal *P. malariae* p33 sequence - p19 from P. malariae - C-terminal *P. knowlesi* p33 sequence - p19 from *P. knowlesi -* p19 from *P. vivax -* C-terminal *P. falciparum* p33 sequence - p19 from *P. falciparum*)** 
MSHHHHHHHHSMSDQVTTGEAESEAPEILVPAGISDYDVVYLKPLAGMYKTIKKQLENHVN
AFNTNITDMLDSRLKKRNYFLEVLNSDLNPFKYSSSGEYIIKDPYKLLDLEKKKKLIGSYKYIG
ASIDMDLATANDGVTYYNKMGELYKTHLDGVKTEIKKVEDDIKKQDEELKKLGNVNSQDSKK
NEFIAKKAELEKYLPFLNSLQKEYESLVSKVNTYTDNLKKVINNCQLEKKEAEITVKKLQDYN
KMDEKLEEYKKSEKKNEVKSSGLLEKLMKSKLIKENESKEILSQLLNVQTQLLAMDESKKLLS
ELLDVDSAQLLSMGSKHKCIDITYPDNAGCYRFSDGREEWRCLLNFKKVGETCVPNNNPTC
AENNGGCDPTADCAESENNKITCTCTGQNESFFEGVFCGNESTKIISELLGVDSNALLNISAK
HACTETKYPENAGCYRYEDGKEVWRCLLNYKLVDGGCVEDEEPSCQVNNGGCAPEANCT
KGDDNKIVCACNAPYSEPIFEGVFCGEEESKKVLSQLLNVQTQMLNMSSAHKCIDTNVPEN
AACYRYLDGTEEWRCLLGFKEVGGKCVPASITCEENNGGCAPEAECTMDDKKEVECKCTK
EGFEPLFEGVFCSTMSSEHTCIDTNVPDNAACYRYLDGTEEWRCLLTFKEEGGKCVPASNV
TCKDNNGGCAPEAECKMTDSNKIVCKCTKEGSEPLFEGVFCSNLAKTVLSNLLDGNLQGML
NISQHQCVKKQCPENSGCFRHLDEREECKCLLNYKQEGDKCVENPNPTCNENNGGCDAD
AKCTEEDSGSNGKKITCECTKPDSYPLFDGIFCS
**SEQ ID NO34 ("His-MSP-multi-His_d" - Purified fusion polypeptide according to the present invention, N-terminally His tagged p33 from *P. vivax* - C-terminal *P***. ***falciparum* p33 sequence - p19 from *P. falciparum* - C-terminal *P. ovale* p33 sequence - p19 from *P. ovale* - C-terminal *P. malariae* p33 sequence - p19 from P. malariae - C-terminal *P. knowlesi* p33 sequence - p19 from *P. knowlesi* - p19 from *P. vivax*)**

MSHHHHHHHHSMSDQVTTGEAESEAPEILVPAGISDYDVVYLKPLAGMYKTIKKQLENHVN
AFNTNITDMLDSRLKKRNYFLEVLNSDLNPFKYSSSGEYIIKDPYKLLDLEKKKKLIGSYKYIG
ASIDMDLATANDGVTYYNKMGELYKTHLDGVKTEIKKVEDDIKKQDEELKKLGNVNSQDSKK
NEFIAKKAELEKYLPFLNSLQKEYESLVSKVNTYTDNLKKVINNCQLEKKEAEITVKKLQDYN
KMDEKLEEYKKSEKKNEVKSSGLLEKLMKSKLIKENESKEILSQLLNVQTQLLAMNLAKTVLS
NLLDGNLQGMLNISQHQCVKKQCPENSGCFRHLDEREECKCLLNYKQEGDKCVENPNPTC

NENNGGCDADAKCTEEDSGSNGKKITCECTKPDSYPLFDGIFCSDESKKLLSELLDVDSAQ
LLSMGSKHKCIDITYPDNAGCYRFSDGREEWRCLLNFKKVGETCVPNNNPTCAENNGGCD
PTADCAESENNKITCTCTGQNESFFEGVFCGNESTKIISELLGVDSNALLNISAKHACTETKY
PENAGCYRYEDGKEVWRCLLNYKLVDGGCVEDEEPSCQVNNGGCAPEANCTKGDDNKIV
CACNAPYSEPIFEGVFCGEEESKKVLSQLLNVQTQMLNMSSAHKCIDTNVPENAACYRYLD
GTEEWRCLLGFKEVGGKCVPASITCEENNGGCAPEAECTMDDKKEVECKCTKEGFEPLFE
GVFCSTMSSEHTCIDTNVPDNAACYRYLDGTEEWRCLLTFKEEGGKCVPASNVTCKDNNG
GCAPEAECKMTDSNKIVCKCTKEGSEPLFEGVFCS

The present invention is further illustrated by the following figures and non-limiting examples from which further features, embodiments, aspects and advantages of the present invention may be taken.

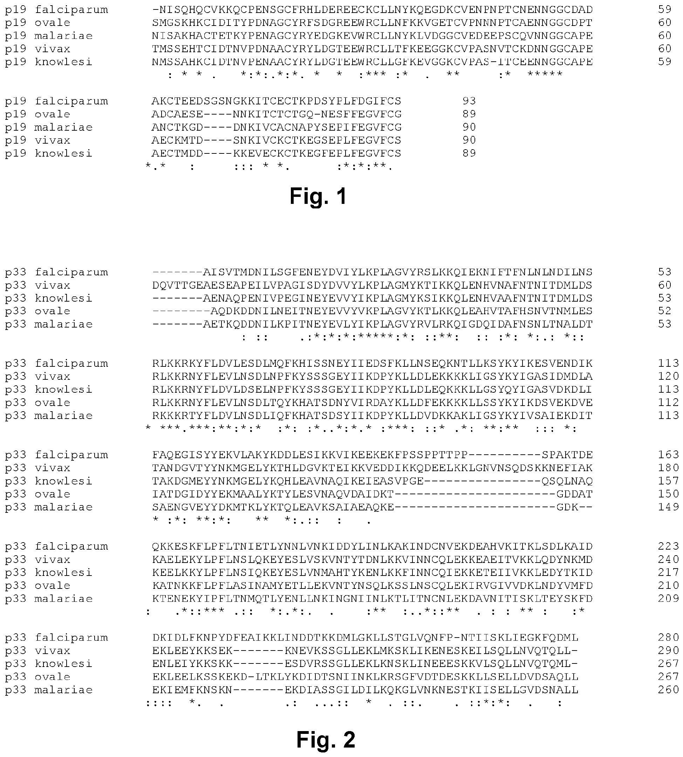

**Fig. 1** shows an alignment of p19 polypeptides from *P. falciparum, P. vivax, P. ovale, P. malariae,* and *P*. *knowlesi.*
**Fig. 2** shows an alignment of p33 polypeptides from *P. falciparum, P. vivax, P. ovale, P. malariae,* and *P. knowlesi.*

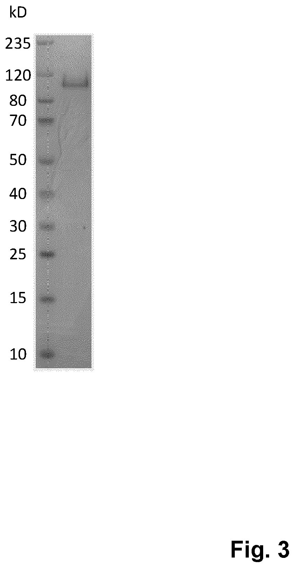

**Fig. 3** shows a molecular weight marker (MW, left) and a purified fusion polypeptide according to the present invention (SEQ ID NO30).

### Example 1:

The following experiments were performed to evaluate the performance of different combinations of *Plasmodium* antigens for the detection of specific anti*-Plasmodium* IgG antibodies in human serum or plasma.

### 1. Samples:

### Panel 1 (sensitivity panel):

The sensitivity panel contained 50 clinical samples of patients with proven *Plasmodium* infection. The causative four *Plasmodium* spp. (*P*. *falciparum* n=26, *P*. *vivax* n=6, *P*. *ovale n=12* and *P*. *malariae* n=6) was determined by microscopy and PCR. Furthermore the clinical samples were analyzed for anti*-Plasmodium* antibodies using the immunofluorescence (IFT) and ELISA.

### Panel 2 (specificity panel):

The specificity panel contains 37 samples which originate from healthy individuals (pregnant woman, children and blood donors from Germany). Previous exposure to *Plasmodium* is highly unlikely because of the origin of the samples.

### 2. Experiments:

### 2.1. Preparation of coated microtiter plates

The following antigens from Merozoite Surface Protein 1 (MSP-1) were used in the form of His-tagged constructs cloned in to vector pET24d (Novagen) and purified by nickel affinity chromatography using standard methodology:
- p19 polypeptide from MSP-1 *P. falciparum* (SEQ ID NO13)
- p19 polypeptide from MSP-1 *P. vivax* (SEQ ID NO15)
- p33 polypeptide from MSP-1 *P. vivax* (SEQ ID NO16)
- p19 polypeptide from MSP-1 *P. ovale* (SEQ ID NO17)
- p19 polypeptide from MSP-1 *P. malariae* (SEQ ID NO18
- p19 polypeptide from MSP-1 *P. knowlesi* (SEQ ID NO19)
- **Fusion polypeptide according to the present invention, N-terminally His tagged, comprising in that order: p33 from *P. vivax*** - **p19 from *P. vivax*** - C-terminal *P. falciparum* p33 sequence - p19 from P. *falciparum* - C-terminal *P*. *ovale* p33 sequence - p19 from *P*. *ovale* - C-terminal P. *malariae* p33 sequence - p19 from P. *malariae* - C-terminal *P*. *knowlesi* p33 sequence - p19 from *P*. *knowlesi* (SEQ ID NO30).

For use in microtiter ELISA these antigens were diluted in PBS to final concentrations of approximately 2.0 µg/ml and used to coat ELISA microtiter plates (Nunc, Roskilde, Denmark).

### 2.2. Experimental procedure

Samples were diluted 1:101 in IgG sample buffer, applied to microtiter plates and incubated as described for commercial EUROIMMUN ELISA Test-Kits (e.g. EI 2260-9601 G). In brief: 60 min at 37 °C; 3 washing steps using EUROIMMUN washing buffer; addition of 100 µl of peroxidase-labelled anti-human IgG conjugate (goat) per well; incubation for 30 min at 37 °C; 3 washing steps using EUROIMMUN washing buffer; addition of 100 µl of chromogen/substrate solution (TMB/H₂O₂) per well; incubation for 30 min at room temperature; addition of 100 µl stop-solution (0.5 M sulfuric acid); measurement of optical density at 450 nm.

Panel 1 was used for evaluation of sensitivity of the respective antigens for detection of specific anti*-Plasmodium* IgG antibodies. Panel 2 was incubated to determine specificity of the test system.

### 3. Results: Sensitivity and specificity of separately expressed p19 and p33 polypeptides of MSP-1 of Plasmodium spp. and a fusion polypeptide comprising them.

Comparison of p19 polypeptides of MSP-1 *P. falciparum, P. vivax, P. ovale, P. malariae* and *P. knowlesi* as well as p33 polypeptides of MSP-1 from *P*. *falciparum* and *P*. *vivax* showed overall sensitivities and specificities of:
The primary data are shown in **Table 1**, a summary of the results in **Table 2**.

**Table 1: Primary data. Columns show the ELISA readouts obtained when the same sample was contacted in separate vessels with individual separately expressed antigens or the fusion polypeptide comprising all of them.**

| No. | **EUROIMMUN Antigen** | | | | | | |
|---|---|---|---|---|---|---|---|
| | ***His-MSP-1-p19*** | ***His-MSP-1- p19*** | ***His-MSP-1- p33*** | ***His-MSP-1- p19*** | ***His-MSP-1- p19*** | ***His-MSP-1- p19*** | ***His-MSP-multi-p19*** |
| | ***P*. *falciparum*** | ***P. vivax*** | ***P. vivax*** | ***P*. *ovale*** | ***P. malariae*** | ***P*. *knowlesi*** | - |
| 1 | 0,266 | **3,203** | **1,001** | 0,250 | **0,402** | **3,025** | **3,263** |
| 2 | 0,294 | **3,207** | **3,243** | 0,069 | 0,273 | **3,120** | **3,246** |
| 3 | 0,139 | **2,370** | **1,025** | 0,063 | 0,159 | **2,359** | **2,769** |
| 4 | 0,161 | **1,863** | **2,654** | 0,131 | 0,116 | **0,480** | **2,626** |
| 5 | 0,137 | **3,325** | **2,957** | **1,212** | 0,223 | **3,198** | **3,386** |
| 6 | 0,252 | **3,122** | **0,499** | **0,529** | 0,313 | **2,684** | **3,342** |
| 7 | 0,330 | 0,181 | 0,144 | 0,144 | 0,164 | 0,104 | **0,680** |
| 8 | 0,202 | 0,069 | 0,193 | 0,097 | 0,109 | 0,065 | 0,220 |
| 9 | **1,101** | 0,199 | **0,706** | 0,134 | 0,182 | 0,117 | **1,774** |
| 10 | **1,060** | 0,258 | **0,510** | 0,181 | 0,180 | 0,165 | **1,823** |
| 11 | **0,836** | 0,129 | 0,276 | 0,139 | 0,135 | 0,126 | **1,632** |
| 12 | 0,223 | 0,217 | 0,229 | 0,331 | 0,293 | 0,282 | 0,186 |
| 13 | 0,205 | 0,228 | 0,246 | 0,238 | 0,210 | 0,196 | 0,167 |
| 14 | **1,159** | 0,320 | 0,270 | 0,318 | **1,700** | 0,398 | **2,321** |
| 15 | 0,263 | 0,233 | 0,328 | 0,227 | 0,245 | 0,205 | **0,438** |
| 16 | **2,236** | **1,148** | **0,946** | **0,663** | **0,613** | **0,959** | **2,797** |
| 17 | **0,506** | 0,357 | **0,730** | 0,306 | 0,267 | **0,808** | **1,503** |
| 18 | **0,527** | 0,333 | **0,713** | 0,360 | 0,306 | **0,847** | **1,372** |
| 19 | **0,886** | 0,222 | **0,418** | **0,420** | 0,253 | 0,223 | **1,926** |
| 20 | 0,337 | **0,541** | **0,808** | 0,232 | 0,273 | 0,376 | **1,689** |
| 21 | **1,171** | 0,287 | **0,518** | **0,571** | 0,311 | 0,280 | **2,208** |
| 22 | **1,071** | **0,764** | **0,754** | **0,510** | 0,397 | **0,706** | **1,814** |
| 23 | **0,978** | 0,220 | 0,344 | 0,214 | 0,250 | 0,171 | **1,902** |
| 24 | **1,057** | **1,118** | **0,766** | **0,590** | **0,660** | **1,142** | **2,597** |
| 25 | 0,147 | 0,165 | 0,132 | 0,187 | 0,176 | 0,225 | 0,194 |
| 26 | 0,222 | 0,271 | 0,182 | 0,275 | 0,247 | 0,292 | 0,213 |
| 27 | **0,749** | **0,579** | 0,184 | 0,176 | 0,167 | **0,437** | **1,648** |
| 28 | 0,248 | 0,204 | 0,272 | 0, 318 | 0,298 | 0,172 | **1,161** |
| 29 | 0,072 | 0,024 | 0,038 | 0,019 | 0,035 | 0,019 | 0,242 |
| 30 | 0,334 | **0,949** | 0,232 | 0,196 | 0,215 | **1,183** | **1,773** |
| 31 | 0,178 | **0,672** | **1,551** | **0,553** | 0,348 | **0,624** | **0,948** |
| 32 | **0,528** | 0,309 | **0,452** | 0362 | 0318 | 0,386 | **0,429** |
| 33 | **1,554** | **3,452** | **3,122** | **1,547** | **2,011** | **3,305** | **3,334** |
| 34 | **0,678** | **0,708** | **0,863** | **1,056** | **0,675** | **0,634** | **1,702** |
| 35 | 0,333 | **0,552** | **0,500** | **1,920** | 0,125 | **0,523** | **2,367** |
| 36 | **1,728** | 0,290 | 0, 381 | **1,693** | 0,250 | 0,257 | **2,815** |
| 37 | 0,194 | 0,216 | 0,138 | **2,106** | 0,170 | 0,183 | **2,844** |
| 38 | **0,873** | 0,349 | 0,232 | **3,121** | **1,661** | 0359 | **3,244** |
| 39 | **0,851** | 0,328 | 0,325 | **3,128** | **1,618** | 0356 | **3,227** |
| **40** | **0,967** | 0,336 | 0,293 | **3,187** | **1,549** | 0355 | **3,302** |
| 41 | **0,602** | **2,641** | 0,385 | **3,120** | 0,296 | **2,067** | **3,421** |
| 42 | **1,141** | 0,237 | 0,250 | **1,076** | **0,420** | 0,220 | **2,612** |
| 43 | 0,188 | 0,095 | 0,248 | 0,186 | 0,102 | 0,087 | **0,683** |
| **44** | 0,141 | 0,091 | 0,142 | 0,155 | 0,356 | 0,101 | **0,410** |
| 45 | **1,146** | **0,821** | **0,819** | **0,722** | **0,653** | **0,706** | **2,323** |
| 46 | **0,942** | 0,338 | 0,282 | 0,272 | **1,500** | 0,188 | **2,799** |
| 47 | **1,709** | **0,641** | **0,899** | **0,714** | **1,837** | **0,606** | **2,591** |
| 48 | **2,147** | **0,655** | **0,864** | **0,772** | **1,974** | **0,681** | **2,533** |
| 49 | **1,835** | **0,636** | **0,849** | **0,652** | **1,702** | **0,609** | **2,722** |
| 50 | **1,691** | 0,156 | 0,214 | **0,443** | 0,298 | 0,126 | **2,263** |
| 51 | 0,153 | 0,074 | 0,066 | 0,062 | 0,085 | 0,107 | 0,108 |
| 52 | 0,086 | 0,045 | 0,034 | 0,043 | 0,052 | 0,051 | 0,060 |
| 53 | 0,115 | 0,065 | 0,061 | 0,065 | 0,062 | 0,064 | 0,077 |
| 54 | 0,078 | 0,033 | 0,050 | 0,025 | 0,041 | 0,040 | 0,045 |
| 55 | 0,095 | 0,066 | 0,076 | 0,059 | 0,049 | 0,058 | 0,069 |
| 56 | 0,185 | 0,092 | 0,084 | 0,061 | 0,074 | 0,118 | 0,158 |
| 57 | 0,154 | 0,106 | 0,062 | 0,078 | 0,100 | 0,110 | 0,088 |
| 58 | 0,168 | 0,057 | 0,239 | 0,050 | 0,060 | 0,077 | 0,246 |
| 59 | 0,231 | 0,102 | 0,055 | 0,067 | 0,092 | 0,124 | 0,128 |
| 60 | 0,199 | 0,085 | 0,056 | 0,052 | 0,071 | 0,095 | 0,134 |
| 61 | 0,134 | 0,127 | 0,087 | 0,100 | 0,094 | 0,131 | 0,135 |
| 62 | 0,301 | **0,751** | 0,200 | 0,291 | 0,233 | **0,540** | 0,166 |
| 63 | 0,193 | 0,070 | 0,071 | 0,083 | 0,063 | 0,070 | 0,117 |
| 64 | 0,031 | 0,041 | 0,075 | 0,026 | 0,038 | 0,040 | 0,124 |
| 65 | 0,032 | 0,019 | 0,030 | 0,021 | 0,032 | 0,023 | 0,028 |
| 66 | 0,024 | 0,013 | 0,036 | 0,017 | 0,012 | 0,016 | 0,031 |
| 67 | 0,033 | 0,026 | 0,333 | 0,026 | 0,032 | 0,035 | 0, 301 |
| 68 | 0,063 | 0,030 | 0,036 | 0,053 | 0,040 | 0,033 | 0,044 |
| 69 | 0,103 | 0,039 | 0,028 | 0,029 | 0,042 | 0,046 | 0,058 |
| 70 | 0,050 | 0,035 | 0,100 | 0,028 | 0,089 | 0,051 | 0,117 |
| 71 | 0,159 | 0,113 | 0,081 | 0,103 | 0,114 | 0,107 | 0,087 |
| 72 | 0,137 | 0,046 | 0,111 | 0,031 | 0,087 | 0,049 | 0,079 |
| 73 | 0,110 | 0,043 | 0,039 | 0,033 | 0,051 | 0,057 | 0,071 |
| 74 | 0,087 | 0,063 | 0,050 | 0,044 | 0,057 | 0,069 | 0,043 |
| 75 | 0,043 | 0,032 | 0,153 | 0,077 | 0,031 | 0,031 | 0,091 |
| 76 | 0,089 | 0,050 | 0,036 | 0,031 | 0,046 | 0,043 | 0,064 |
| 77 | 0,128 | 0,043 | 0,118 | 0,037 | 0,044 | 0,055 | 0,090 |
| 78 | 0,097 | 0,047 | 0,039 | 0,043 | 0,062 | 0,071 | 0,045 |
| 79 | 0,253 | 0,192 | 0,163 | 0,214 | 0,195 | 0,188 | 0,146 |
| 80 | 0,124 | 0,081 | 0,065 | 0,075 | 0,082 | 0,069 | 0,054 |
| 81 | 0,157 | 0,044 | 0,036 | 0,041 | 0,048 | 0,058 | 0,100 |
| 82 | 0,171 | 0,079 | 0,046 | 0,038 | 0,067 | 0,096 | 0,147 |
| 83 | 0,232 | 0,096 | 0,059 | 0,071 | 0,107 | 0,112 | 0,140 |
| 84 | 0,266 | 0,164 | 0,091 | 0,115 | 0,128 | 0,146 | 0,141 |
| 85 | 0,064 | 0,032 | 0,025 | 0,029 | 0,049 | 0,033 | 0,032 |
| 86 | 0,126 | 0,060 | 0,113 | 0,051 | 0,116 | 0,063 | 0,107 |
| 87 | 0,193 | 0,102 | 0,108 | 0,119 | 0,142 | 0,113 | 0,133 |

**Table 2: Summary of results**

| Antibodies detected against | Total sensitivity in % | Total specificity in % |
|---|---|---|
| p19 from *falciparum, vivax, ovale, malariae* and *knowlesi and p33 from P. vivax, each expressed* as *separate N-terminally His-tagged protein* | 39/50 | 36/37 |
| | 78% | 97,3% |
| Fusion protein according to the present invention (SEQ ID NO30) | 44/50 | 37/37 |
| | 88% | 100% |

The sample set used for evaluation of sensitivity of this test are samples originating from patients with acute infections. If these samples were drawn at early time points of infection there would be the possibility that they lack anti*-Plasmodium* IgG because seroconversion has yet not taken place (four samples positive for PCR and microscopy, but serology is negative.

### Conclusion

First of all, the intensity of the signal is considerably increased if the fusion polypeptide is used rather than separately expressed antigens, even if an antibody to one antigen is present only, for example in samples 9 to 11, wherein antibodies to *P*. *falciparum* p19 polypeptide are present only.

Secondly, it is clear that the signal is above the cutoff in a larger number of samples from patients suffering from Malaria, showing the increased sensitivity of the assay. The specificity is only slightly better.

### Example 2:

Additional fusion polypeptides comprising **p19 and p33 polypeptides of MSP-1 of *Plasmodium* spp.** were used for the detection of antibodies in human blood samples. Experiments were essentially carried out as described in Example 1, but using additional polypeptides according to the present invention.

### Panel 1 (sensitivity panel):

The sensitivity panel contains 47 clinical samples of patients with proven Plasmodium infection. The causative four Plasmodium spp. (P. falciparum n=24, P. vivax n=5, P. ovale n=12 and P. malariae n=6) was determined by microscopy and PCR. Furthermore the clinical samples were analysed for anti-Plasmodium antibodies via IFT and ELISA.

The panel overlapped with Panel 1 used in Experiment 1 with the exception that few samples could not be examined again because the quantities available were insufficient. The numbers of the samples are identical in Panels 1 and 2.

### Panel 2 (specificity panel):

The specificity panel contains 16 samples which originate from healthy individuals (pregnant woman, children and blood donors from Germany). It can be assumed that the donors had not been exposed to Plasmodium.

### 1. Preparation of coated microtiter plates

The following antigens from Merozoite Surface Protein 1 (MSP-1) were used:
- fusion protein from p19 polypeptides of MSP-1 P. falciparum, P. vivax, P. ovale, P. malariae and P. knowlesi (MSP-1 multi-p19) (SEQ ID NO30)
- His-MSP-1-multi_a (SEQ ID NO31)
- His-MSP-1-multi_b (SEQ ID NO32)
- His-MSP-1-multi_c (SEQ ID NO33)
- His-MSP-1-multi_d (SEQ ID NO34)

For use in microtiter ELISA these antigens were diluted in PBS to final concentrations of 5,0 µg/ml. Microtiter plates were coated with 100 µl of antigen dilution per well.

### 2. Experimental procedure

All reagents used during this experiment are included in every EUROIMMUN IgG ELISA Test-Kit for infectious diagnostics. Sample were diluted 1:101 in IgG sample buffer, were applied to microtiter plates and incubated as described for commercial EUROIMMUN ELISA Test-Kits (e.g. EI 2260-9601 G). In brief: 60 min at 37 °C; 3 washing steps using EUROIMMUN wash buffer; addition of 100 µl of peroxidase-labelled anti-human IgG conjugate (goat) per well; incubation for 30 min at 37 °C; 3 washing steps using EUROIMMUN wash buffer; addition of 100 µl of chromogen/substrate solution (TMB/H2O2) per well; incubation for 30 min at room temperature; addition of 100 µl stop-solution (0.5 M sulfuric acid); measurement of optical density at 450 nm.

Panel 1 was used for evaluation of sensitivity of the respective antigens for detection o specific anti-Plasmodium IgG antibodies. Panel 2 was incubated to determine specificity of the test system.

### 3. Results

The primary data are shown in **Table 3.** Five samples that could be correctly identified as positive using the polypeptides according to the invention, but not separately expressed polypeptides **in Example 1** are **in bold.** "no Mat." Indicates that the samples were examined only in Example 1, but not again in Example 2, because the volumes of the sample were insufficient. Samples 1 to 6 are from patients exposed to P. vivax. Samples 7 to 32 are from patients exposed to P. falciparum. Samples 33 to 44 are from patients exposed to P. ovale. Samples 45 to 50 are from patients exposed to P. malariae. Samples 51 to 58 are positive controls. The remaining samples are from healthy blood donors.

| **No**. | ***MSP1_multi-His_a*** -5,0 µg/ml **pos:≥ 0,400 OD** bl: 0.300-0,399 OD | ***MSP1_Multi- His*_*b-***5,0 µg/ml **pos:≥ 0,400 OD** bl: 0.300-0,399 OD | ***MSP1_multi*- *His_c*** 5,0 µg/ml **pos:≥ 0,400 OD** bl: 0.300-0,399OD | ***MSP1_multi-His_d*** -5,0 µc/ml **pos:≥ 0,400 OD** bl: 0.300-0,399 OD | ***His-MSP-multi-p19*** -5,0 µg/mL **pos:**≥ **0,400 OD** bl: 0.300-0,399 OD |
|---|---|---|---|---|---|
| 1 | 3,381 | 3,467 | 3,418 | 3,356 | 3,330 |
| 2 | 3,397 | 3,451 | 3,262 | 3,308 | 3,329 |
| 3 | no Mat | no Mat | no. Mat | no. Mat | no. Mat |
| 4 | 2,830 | 2,717 | 2,050 | 2,292 | 2,593 |
| 5 | 3,523 | 3,621 | 3,504 | 3,449 | 3,426 |
| 6 | 3,463 | 3,538 | 3,501 | 3,464 | 3,432 |
| **7** | **0,844** | **0,849** | **0,746** | **0,754** | **0,724** |
| 8 | 0,385 | 0,347 | 0,313 | 0,249 | 0,213 |
| 9 | no Mat | no Mat | no. Mat | no. Mat | no. Mat |
| 10 | 2,778 | 2,945 | 2,881 | 2,906 | 1,799 |
| 11 | 2,720 | 2,912 | 2,749 | 2,760 | 1,596 |
| 12 | 0,151 | 0,133 | 0,184 | 0,146 | 0,146 |
| 13 | 0,115 | 0,126 | 0,163 | 0,130 | 0,129 |
| 14 | 2,930 | 3,108 | 2,964 | 2,526 | 2,796 |
| **15** | **1,227** | **1,032** | **0,930** | **0,865** | **0,431** |
| 16 | 3,548 | 3, 349 | 3,274 | 3,187 | 2,915 |
| 17 | no Mat | no Mat | no. Mat | no. Mat | no. Mat |
| 18 | 2,482 | 2,499 | 2,308 | 2,291 | 1,499 |
| 19 | 2,289 | 2,323 | 2,162 | 2,058 | 1,831 |
| 20 | 2,755 | 3,003 | 2,759 | 2,685 | 1,725 |
| 21 | 2,813 | 2,860 | 2,634 | 2,576 | 2,236 |
| 22 | 3,097 | 3,308 | 3,002 | 3,129 | 2,157 |
| 23 | 2,937 | 3,115 | 2,933 | 3,022 | 1,774 |
| 24 | 3,212 | 3,229 | 3,089 | 3,084 | 2,701 |
| **25** | 0,219 | 0,207 | 0,306 | 0,164 | 0,187 |
| **26** | 0,307 | 0,276 | **0,494** | 0,233 | 0,242 |
| 27 | 2,333 | 2,450 | 2,284 | 2,157 | 1,670 |
| **28** | **1,625** | **1,824** | **1,618** | **1,598** | **1,151** |
| 29 | 0,275 | 0,285 | 0,270 | 0,265 | 0,235 |
| 30 | 2,229 | 2,626 | 2,331 | 2,298 | 2,029 |
| 31 | 2,126 | 2,179 | 1,916 | 1,884 | 1,073 |
| 32 | 0,521 | 0,498 | 0,441 | 0,432 | 0,415 |
| 33 | 3,683 | 3,646 | 3,688 | 3,656 | 3,502 |
| 34 | 1,730 | 2,115 | 1,556 | 1,960 | 1,649 |
| 35 | 2,570 | 2,676 | 2,414 | 2,421 | 2,358 |
| 36 | 3,309 | 3,333 | 3,321 | 3,303 | 2,899 |
| 37 | 3,077 | 3,217 | 3,148 | 3,271 | 3,022 |
| 38 | 3,553 | 3,600 | 3,635 | 3,575 | 3,493 |
| 39 | 3,452 | 3,585 | 3,603 | 3,562 | 3,388 |
| 40 | 3,598 | 3,627 | 3,590 | 3,712 | 3,578 |
| 41 | 3,667 | 3,677 | 3,628 | 3,553 | 3,523 |
| 42 | 2,926 | 2,993 | 2,805 | 2,692 | 2,604 |
| **43** | **0,772** | **1,114** | **0,941** | **1,801** | **0,699** |
| **44** | 0,357 | **0,743** | **0,721** | **1,310** | 0,396 |
| 45 | 3,082 | 3,185 | 3,000 | 2,935 | 2,325 |
| 46 | 3,196 | 3,347 | 3,164 | 3,139 | 2,795 |
| 47, | 2,683 | 3,158 | 2,866 | 2,929 | 2,668 |
| 48 | 2,828 | 3,274 | 2,876 | 2,965 | 2,833 |
| 49 | 2,776 | 3,019 | 2,812 | 2,965 | 2,625 |
| 50 | 2,885 | 2,906 | 2,856 | 2,679 | 2,247 |
| 51 | 0,989 | 1,057 | 1,055 | 0,795 | 0,419 |
| 52 | 1,460 | 1,459 | 1,425 | 1,330 | 0,829 |
| 53 | 1,300 | 1,285 | 1,246 | 1,195 | 1,240 |
| 54 | 1,913 | 2,090 | 1,985 | 1,754 | 1,438 |
| 55 | 1,066 | 1,313 | 1,110 | 1,035 | 0,692 |
| 56 | 1,435 | 1,502 | 1,211 | 1,358 | 0,794 |
| 57 | 1,319 | 1,274 | 0,627 | 1,122 | 1,522 |
| 58 | 3,158 | 3,284 | 3,107 | 3,170 | 2,637 |
| 59 | 0,150 | 0,104 | 0,078 | 0,138 | 0,166 |
| 60 | 0,082 | 0,067 | 0,097 | 0,126 | 0,100 |
| 61 | 0,192 | 0,068 | 0,083 | 0,124 | 0,145 |
| 62 | 0,090 | 0,089 | 0,102 | 0,118 | 0,131 |
| 63 | 0,223 | 0,177 | 0,147 | 0,158 | 0,166 |
| 64 | 0,074 | 0,067 | 0,173 | 0,048 | 0,109 |
| 65 | 0,062 | 0,043 | 0,045 | 0,043 | 0,055 |
| 66 | 0,167 | 0,119 | 0,061 | 0,082 | 0,096 |
| 67 | 0,041 | 0,062 | 0,052 | 0,072 | 0,058 |
| 68 | 0,164 | 0,150 | 0,081 | 0,154 | 0,186 |
| 69 | 0,135 | 0,128 | 0,084 | 0,147 | 0,164 |
| 70 | 0,136 | 0,101 | 0,113 | 0,174 | 0,193 |
| 71 | 0,096 | 0,091 | 0,103 | 0,127 | 0,149 |
| 72 | 0,035 | 0,036 | 0,029 | 0,029 | 0,067 |
| 73 | 0,147 | 0,133 | 0,095 | 0,117 | 0,162 |
| 74 | 0,114 | 0,098 | 0,110 | 0,147 | 0,157 |

The results are summarized in **Table 4:**

| Antibodies detected against | Total sensitivity in % | Total specificity in % |
|---|---|---|
| p19 from *falciparum, vivax, ovale, malariae* and *knowlesi and p33 from P. vivax,* each expressed as separate N-terminally His-tagged protein (taken from Example 1) | 39/50 | 36/37 |
| | 78% | 97,3% |
| Fusion protein MSP-1 multi-p19 according to the present invention (SEQ ID NO30) | 40/46 | 16/16 |
| | 87% | 100% |
| Fusion protein His-MSP-1-multi_a according to the present invention (SEQ ID NO31) | 40/44 | 16/16 |
| | 91% | 100% |
| Fusion protein His-MSP-1-multi_b according to the present invention (SEQ ID NO32) | 41/46 | 16/16 |
| | 88% | 100% |
| Fusion protein His-MSP-1-multi_c according to the present invention (SEQ ID NO33) | 42/45 | 16/16 |
| | 93% | 100% |
| Fusion protein His-MSP-1-multi_d according to the present invention (SEQ ID NO34) | 41/47 | 16/16 |
| | 87% | 100% |

Borderline results were not included in the calculation of sensitivity.

Interestingly, the five samples shown to be positive using the polypeptide according to the invention appeared to be positive no matter independent of the order of the antigen components, with the exception of sample 41 which was positive only in the case of three of the five constructs. However, the values obtained using the other two constructs were borderline and quite close to the cut off value.

All tested MSP-1 polypeptides reveals a high specificity.

### 3.1. Conclusion

Our results show that the order of the p19 fragments has no significant impact on sensitivity and specificity of the MSP-1 multi-p19 antigen. Regardless of the order, the sensitivity is increased at a high level, while the specificity is not compromised.

## Claims

1. A polypeptide comprising four or five p19 polypeptides, preferably all, from the group comprising
p19 from *P. falciparum* according to SEQ ID NO2,
p19 from *P*. *vivax* according to SEQ ID NO3,
p19 from *P*. *ovale* according to SEQ ID NO4,
p19 from *P. malariae* according to SEQ ID NO5 and
p19 from *P*. *knowlesi* according to SEQ ID NO6 and
further comprises a p33 polypeptide from a *Plasmodium* strain according to SEQ ID NO7,
wherein all comprised polypeptides are covalently linked in said polypeptide that comprises the entirety of the polypeptides.

2. The polypeptide according to claim 1, wherein the polypeptide is immobilized, preferably on a diagnostically useful carrier.

3. The polypeptide according to any of claims 1 to 2, wherein the polypeptide is purified.

4. A diagnostically useful carrier coated with the polypeptide according any of claims 1 to 3, preferably immobilized directly on the carrier, wherein the carrier is preferably selected from the group comprising a glass slide, preferably for microscopy, a biochip, a microtiter plate, a lateral flow device, a test strip, a membrane, preferably a line blot, a chromatography column and a bead, preferably a magnetic or fluorescent bead.

5. An *in-vitro* method comprising the step detecting in a liquid four or five antibodies, preferably all, from the group comprising
an antibody to p19 from *P*. *falciparum,*
an antibody to p19 from *P*. *vivax,*
an antibody to p19 from *P*. *ovale,*
an antibody to p19 from *P*. *malariae* and
an antibody to p19 from *P*. *knowlesi,*
comprising the step contacting the liquid with the polypeptide or carrier according to any of claims 1 to 4,
wherein in addition an antibody to a p33 polypeptide from a *Plasmodium* strain is detected.

6. The method according to any of claim 5, wherein the antibody is detected using a method selected from the group comprising immunodiffusion techniques, immunoelectrophoretic techniques, light scattering immunoassays, agglutination techniques, labeled immunoassays such as those from the group comprising radiolabeled immunoassay, enzyme immunoassays, more preferably ELISA, chemiluminscence immunoassays, preferably electrochemiluminescence immunoassay, and immunofluorescence, preferably indirect immunofluorescence.

7. A method comprising the step coating an analytically useful carrier with the polypeptide according to any of claims 1 to 3, wherein preferably the carrier is selected from the group comprising a glass slide, preferably for microscopy, a biochip, a microtiter plate, a lateral flow device, a test strip, a membrane, preferably a line blot, a chromatography column and a bead, preferably a magnetic or fluorescent bead.

8. An *in-vitro* use of the polypeptide or diagnostically useful carrier according to any of claims 1 to 4 for identifying blood from a non-infected blood donor.

9. The method or use according to any of claims 5, 6 or 8, wherein one or more antibody is detected in a processed fraction of blood, preferably from a blood donor.

10. A kit comprising the polypeptide or carrier according to any of claims 1 to 4, further comprising one or more reagents from the group comprising one or more than one calibrator, a washing buffer and a means for detecting an antibody, preferably a labeled secondary antibody.

11. A use of the polypeptide according to claim 1 to 3 for the manufacture of an analytical device, or for the manufacture of a composition, kit or reagent for detecting a *Plasmodium* infection.

12. A method for determining the ability, preferably capacity of a carrier according to claim 4 to bind two or more antibodies to *Plasmodium* p19 proteins, comprising the step contacting the diagnostically useful carrier according to claim 4 with one or more solutions comprising known concentrations of the two or more antibodies.

## Patentansprüche

1. Polypeptid umfassend vier oder fünf p19-Polypeptide, vorzugsweise alle, aus der Gruppe umfassend
p19 aus *P*. *falciparum* gemäß SEQ ID NO2,
p19 aus *P*. *vivax* gemäß SEQ ID NO3,
p19 aus *P*. *ovale* gemäß SEQ ID NO4,
p19 aus *P*. *malariae* gemäß SEQ ID NO5 und
p19 aus *P*. *knowlesi* gemäß SEQ ID NO6 und
ferner umfassend ein p33-Polypeptid aus einem *Plasmodium-*Stamm gemäß SEQ ID NO7,
wobei alle umfassten Polypeptide kovalent in dem Polypeptid, das die Gesamtheit der Polypeptide umfasst, verbunden sind.

2. Polypeptid nach Anspruch 1, wobei das Polypeptid immobilisiert ist, vorzugsweise auf einem diagnostisch geeigneten Träger.

3. Polypeptid nach einem der Ansprüche 1 bis 2, wobei das Polypeptid gereinigt ist.

4. Diagnostisch geeigneter Träger, beschichtet mit dem Polypeptid nach einem der Ansprüche 1 bis 3, vorzugsweise direkt auf dem Träger immobilisiert, wobei der Träger vorzugsweise ausgewählt ist aus der Gruppe, die einen Objektträger, vorzugsweise für die Mikroskopie, einen Biochip, eine Mikrotiterplatte, eine Lateral-Flow-Vorrichtung, einen Teststreifen, eine Membran, vorzugsweise einen Line-Blot, eine Chromatographiesäule und ein Bead, vorzugsweise ein magnetisches oder fluoreszierendes Bead, umfasst.

5. *In-vitro*-Verfahren umfassend den Schritt Nachweisen in einer Flüssigkeit von vier oder fünf Antikörpern, vorzugsweise allen, aus der Gruppe umfassend
einen Antikörper gegen p19 aus *P*. *falciparum,*
einen Antikörper gegen p19 aus *P*. *vivax,*
einen Antikörper gegen p19 aus *P*. *ovale,*
einen Antikörper gegen p19 aus *P*. *malariae* und
einen Antikörper gegen p19 aus *P*. *knowlesi,*
umfassend den Schritt Inkontaktbringen der Flüssigkeit mit dem Polypeptid oder Träger nach einem der Ansprüche 1 bis 4,
wobei zusätzlich ein Antikörper gegen ein p33-Polypeptid aus einem *Plasmodium-*Stamm nachgewiesen wird.

6. Verfahren nach Anspruch 5, wobei der Antikörper unter Verwendung eines Verfahrens nachgewiesen wird, das aus der Gruppe umfassend Immundiffusionsverfahren, immunelektrophoretische Verfahren, Lichtstreuungsimmunassays, Agglutinationsverfahren, markierte Immunassays, wie z.B. solche aus der Gruppe umfassend radioaktiv markierte Immunassays, Enzym-Immunassays, besonders bevorzugt ELISA, Chemilumineszenz-Immunassays, vorzugsweise Elektrochemilumineszenz-Immunassay, und Immunfluoreszenz, vorzugsweise indirekte Immunfluoreszenz, ausgewählt ist.

7. Verfahren umfassend den Schritt Beschichten eines analytisch geeigneten Trägers mit dem Polypeptid nach einem der Ansprüche 1 bis 3, wobei der Träger vorzugsweise aus der Gruppe umfassend einen Objektträger, vorzugsweise für die Mikroskopie, einen Biochip, eine Mikrotiterplatte, eine Lateral-Flow-Vorrichtung, einen Teststreifen, eine Membran, vorzugsweise einen Line-Blot, eine Chromatographiesäule und ein Bead, vorzugsweise ein magnetisches oder fluoreszierendes Bead, ausgewählt ist.

8. *In-vitro*-Verwendung des Polypeptids oder des diagnostisch geeigneten Trägers nach einem der Ansprüche 1 bis 4 zum Identifizieren von Blut von einem nicht infizierten Blutspender.

9. Verfahren oder Verwendung nach einem der Ansprüche 5, 6 oder 8, wobei ein oder mehrere Antikörper in einer verarbeiteten Blutfraktion, vorzugsweise von einem Blutspender, nachgewiesen werden.

10. Kit umfassend das Polypeptid oder den Träger nach einem der Ansprüche 1 bis 4, ferner umfassend ein oder mehrere Reagenzien aus der Gruppe, die einen oder mehr als einen Kalibrator, einen Waschpuffer und ein Mittel zum Nachweisen eines Antikörpers, vorzugsweise einen markierten Sekundärantikörper, umfasst.

11. Verwendung des Polypeptids nach einem der Ansprüche 1 bis 3 für die Herstellung einer analytischen Vorrichtung oder für die Herstellung einer Zusammensetzung, eines Kits oder eines Reagens zum Nachweisen einer *Plasmodium-*Infektion*.*

12. Verfahren zum Bestimmen der Fähigkeit, vorzugsweise der Kapazität, eines Trägers nach Anspruch 4, zwei oder mehr Antikörper an *Plasmodium-*p19-Proteine zu binden, umfassend den Schritt Inkontaktbringen des diagnostisch geeigneten Trägers nach Anspruch 4 mit einer oder mehreren Lösungen, die bekannte Konzentrationen der zwei oder mehr Antikörper umfassen.

## Revendications

1. Polypeptide comprenant quatre ou cinq polypeptides p19, préférablement tous, du groupe comprenant
p19 de *P*. *falciparum* selon la SEQ ID NO2,
p19 de *P*. *vivax* selon la SEQ ID NO3,
p19 de *P*. *ovale* selon la SEQ ID NO4,
p19 de *P*. *malariae* selon la SEQ ID NO5 et
p19 de *P*. *knowlesi* selon la SEQ ID NO6 et
comprenant en outre un polypeptide p33 d'une souche de *Plasmodium* selon la SEQ ID NO7,
tous les polypeptides compris étant liés de manière covalente dans ledit polypeptide qui comprend l'entièreté des polypeptides.

2. Polypeptides selon la revendication 1, le polypeptide étant immobilisé, préférablement sur un support utile pour un diagnostic.

3. Polypeptides selon l'une quelconque des revendications 1 à 2, le polypeptide étant purifié.

4. Support utile pour un diagnostic revêtu par le polypeptide selon l'une quelconque des revendications 1 à 3, préférablement immobilisé directement sur le support, le support étant préférablement choisi dans le groupe constitué par une lame de verre, préférablement pour microscopie, une biopuce, une plaque de microtitration, un dispositif à écoulement latéral, une bande de test, une membrane, préférablement une membrane de transfert en ligne, une colonne de chromatographie et une bille, préférablement une bille magnétique ou fluorescente.

5. Procédé *in vitro* comprenant l'étape de détection dans un liquide de quatre ou cinq anticorps, préférablement tous, du groupe comprenant
un anticorps dirigé contre p19 de *P*. *falciparum,*
un anticorps dirigé contre p19 de *P*. *vivax,*
un anticorps dirigé contre p19 de *P*. *ovale,*
un anticorps dirigé contre p19 de *P*. *malariae* et
un anticorps dirigé contre p19 de *P*. *knowlesi,*
comprenant l'étape de mise en contact du liquide avec le polypeptide ou le support selon l'une quelconque des revendications 1 à 4,
de plus, un anticorps dirigé contre un polypeptide p33 d'une souche de *Plasmodium* étant détecté.

6. Procédé selon la revendication 5, l'anticorps étant détecté en utilisant un procédé choisi dans le groupe comprenant des techniques d'immunodiffusion, des techniques immunoélectrophorétiques, des dosages immunologiques de diffusion de lumière, des techniques d'agglutination, des dosages immunologiques marqués tels que ceux du groupe comprenant un dosage immunologique radiomarqué, des dosages immunologiques enzymatiques, préférablement ELISA, des dosages immunologiques par chimioluminescence, préférablement un dosage immunologique par électrochimioluminescence, et l'immunofluorescence, préférablement l'immunofluorescence indirecte.

7. Procédé comprenant l'étape de revêtement d'un support analytiquement utile par le polypeptide selon l'une quelconque des revendications 1 à 3, préférablement le support étant choisi dans le groupe constitué par une lame de verre, préférablement pour microscopie, une biopuce, une plaque de microtitration, un dispositif à écoulement latéral, une bande de test, une membrane, préférablement une membrane de transfert en ligne, une colonne de chromatographie et une bille, préférablement une bille magnétique ou fluorescente.

8. Utilisation *in vitro* du polypeptide ou du support utile pour un diagnostic selon l'une quelconque des revendications 1 à 4 pour l'identification de sang provenant d'un donneur de sang non infecté.

9. Procédé ou utilisation selon l'une quelconque des revendications 5, 6 et 8, un ou plusieurs anticorps étant détectés dans une fraction de sang traitée, préférablement provenant d'un donneur de sang.

10. Kit comprenant le polypeptide ou le support selon l'une quelconque des revendications 1 à 4, comprenant en outre un ou plusieurs réactifs du groupe comprenant un ou plus d'un calibrateur, un tampon de lavage et un moyen pour détecter un anticorps, préférablement un anticorps secondaire marqué.

11. Utilisation du polypeptide selon les revendications 1 à 3 pour la fabrication d'un dispositif analytique, ou pour la fabrication d'une composition, d'un kit ou d'un réactif pour détecter une infection par *Plasmodium.*

12. Procédé pour la détermination de l'aptitude, préférablement de la capacité d'un support selon la revendication 4 à se lier à deux anticorps ou plus dirigés contre des protéines p19 de *Plasmodium,* comprenant l'étape de mise en contact du support utile pour un diagnostic selon la revendication 4 avec une ou plusieurs solutions comprenant des concentrations connues des deux anticorps ou plus.
